# EUROPEAN PATENT APPLICATION

(11) **EP 3 633 009 A1**
(43) Date of publication of application: **08.04.2020**
(21) Application number: 18199451.8
(22) Date of filing: 09.10.2018
(51) Int. Cl.: C09K 11/06, G01N 21/62

(54) **USE OF A SUBSTITUTED OR UNSUBSTITUTED POLYCYCLIC AROMATIC HYDROCARBON COMPOUND FOR HIGH-RESOLUTION MICROSCOPY**

(30) Priority: 03.10.2018 EP 18198494
(71) Applicant: Max-Planck-Gesellschaft zur Förderung der Wissenschaften e.V., 80539 München (DE)
(72) Inventor: LIU, Xiaomin, 55118 Maintz (DE); NARITA, Akimitsu, 55127 Mainz (DE); PAREKH, Sapun, Austin, Texas 78757 (US); CHEN, Qiang, 55124 Mainz (DE); MÜLLEN, Klaus, 50939 Köln (DE); CREMER, Christoph, 69126 Heidelberg (DE); LANDFESTER, Katharina, 55122 Mainz (DE); BONN, Mischa, 60318 Frankfurt am Main (DE)
(74) Representative: Henkel & Partner mbB

(57) **Abstract**

The present invention relates to the use of a compound in single-molecule localization microscopy (SMLM) or in stimulated emission depletion microscopy (STED), wherein the compound is a substituted or unsubstituted polycyclic aromatic hydrocarbon comprising six or more substituted and/or unsubstituted aromatic hydrocarbon rings, wherein each of at least six of the six or more substituted and/or unsubstituted aromatic hydrocarbon rings is fused with at least another one of the at least six substituted and/or unsubstituted aromatic hydrocarbon rings.

## Description

The present invention relates to the use of a compound for two specific kinds of super-resolution microscopy techniques, namely for single-molecule localization microscopy (SMLM) or stimulated emission depletion microscopy (STED).

Super-resolution microscopy denotes microscopy techniques, which have a higher resolution than the diffraction limit of light, i.e. which have a higher lateral resolution than λ/(2 N.A.), wherein N.A. is the numerical aperture and λ is the wavelength of the light used.

Prominent examples for super-resolution microscopy techniques are single-molecule localization microscopy (SMLM) and stimulated emission depletion microscopy (STED).

The SMLM techniques base on the use of fluorophores, i.e. fluorescent chemical compounds that emit light after having been excited by the absorption of radiation, as markers, which have a low duty cycle. Fluorophores with a low duty cycle are characterized in that - when radiated with excitation radiation - the ratio of the period of time during which they are in the "on" state (i.e. radiation emitting state) divided by the period of time during which they are in the "off" state (i.e. non-emissive ground state or "dark state", respectively) is low. In other words, the used fluorophores show - seen over the time - a fluorescence emission spectrum with emission peaks having a short peak width on the time axis, wherein the time interval between two emission peaks is comparable long. A fluorophore having a low duty cycle is also denoted to have good blinking properties. Therefore, when a sample of which parts are labelled with the respective fluorophore molecules is excited by a laser, such as when a cell of which the microtubules are labelled with the respective fluorophore molecules is excited by a laser, at every point of time only a comparable small number of the fluorophore molecules is in the "on" state and emits fluorescence radiation for a comparable short time period. Thus, the probability that adjacent fluorophore molecules emit radiation at the same point of time is quite low so that consequently the risk of an overlap of the emission signals of two adjacent fluorophore molecules is very low. On account of this reason, a spatial and temporal separation of the fluorescence emission profiles of the single fluorophore molecules is obtained, which allows to precisely reconstruct the position of single fluorophores by mapping the (usually many thousands) pictures obtained over the measurement time.

Apart from good blinking properties, fluorophores suitable for SMLM shall provide further properties. Important is particularly that the fluorophore has a high photon number, i.e. that it emits a high number of photons during its short "on" states. Furthermore, it is preferred that the fluorophore has a very high photostability so that it can be cycled between the "on" state and "off" state as often as possible and thus does not photobleach within short term, i.e. does loose its ability to be excited into the "on" state after a low number of cycles between the "on" state and "off" state. Apart from that, the fluorophore shall have a small size of below 5 nm and ideally of below 1 nm (in order to allow an excellent spatial resolution), a comparable narrow excitation spectrum and shall emit visible light, in order to allow to obtain an excellent spatial separation of the fluorescence emission profiles of the single fluorophore molecules. Particularly preferably, the fluorophore shall have a low toxicity.

Organic dyes, such as Alexa Fluor® 647 dye distributed from ThermoFisher Scientific, show good blinking properties, a narrow excitation spectrum as well as a narrow emission spectrum. However, a special buffer is needed, such as an oxygen-depleted environment in combination with redox agents, in order to maintain these properties. This makes the process not only laborious, but makes it in particular challenging to perform a high-quality imaging in all environments. Moreover, such dyes have good blinking behaviours only within several hours, such as e.g. less than 8 hours, since the special buffer condition changes overtime.

Also known for the purpose of being used as fluorophore for SMLM are so called carbon dots (CDs), which are supposed to be composed of more or less spherical-shaped amorphous carbon. CDs are normally synthesized from carbon soot or carbon black. This is a so-called "top-down" method, i.e. a method, in which a comparable large structure, namely carbon soot or carbon black, is disintegrated into a smaller structure, namely CDs. For instance, carbon black is refluxed with nitric acid for 24 hours, before the resultant suspension is cooled to room temperature and then centrifuged. After discard of the pellet, the supernatant is heated and dried. The so obtained solid is resuspended in water and ultrafiltrated. Carbon dots as well as their preparation methods are disclosed e.g. by Lemenager et al., "Super-resolution fluorescence imaging of biocompatible carbon dots", Nanoscale, 2014, volume 6, pages 8617 to 8623, by He et al., "High-density super-resolution localization imaging with blinking carbon dots", Anal. Chem. 2017, volume 89, pages 11831 to 11838, by Khan et al., "reversible photoswitching of carbon dots", Scientific Reports, 2015, volume 5, pages 1 to 7, and by Verma et al., "Single-molecule analysis of fluorescent carbon dots towards localization-based super-resolution microscopy", 2016, volume 4, pages 1 to 10. However, the CDs are quite large having dimensions of at least 3 nm up to 60 nm and thus too large to obtain a very good resolution with SMLM. Moreover, due to the "top-down" production method, CDs are a mixture of a variety of differently oxidized molecules, which are only sorted according to their size by centrifugation or membrane filters with different pore sizes. On account of this reason CDs are heterogeneous both in size and structure and they are oxidized to variable extents. Thus, CDs do not behave like material being composed of one specific type of molecule. On account of their different species and their heterogeneity in size, the emission spectra and in particular the excitation spectra of CDs are quite broad. Furthermore, due to their undefined chemical structure they cannot be used in targeted intracellular delivery and for specific labelling of subcellular targets.

Compositions being similar to CDs are graphene quantum dots (GQDs). GQDs should be monolayer nanographene by definition, but, however, are often stacked multilayer graphite mixtures containing structures and bear many different oxygen-based functional groups, which derive from their synthesis. Likewise to CDs, GQDs are normally synthesized from carbon soot or carbon black by a "top-down" method. GQDs as well as their preparation methods are disclosed e.g. by Muthurasu et al., "Facile and simultaneous synthesis of graphene quantum dots and reduced graphene oxide for bio-imaging and supercapacitor applications", New. J. Chem., 2016, volume 40, pages 9111 to 9124, by Sarkar et al., "Graphene quantum dots from graphite by liquid exfoliation showing excitation-independent emission, fluorescence upconversion and delayed fluorescence", Phys. Chem. Chem. Phys., 2016, volume 18, pages 21278 to 21287, and by Gan et al., "Mechanism for excitation-dependent photoluminescence from graphene quantum dots and other graphene oxide derivatives: consensus, debates and challenges", Nanoscale, 2016, volume 8, pages 7794 to 7807. On account of being mixtures of single species being heterogeneous both in size and structure, the emission spectra as well as in particular their excitation spectra are quite broad. In particular GQDs have a low quantum yield and the broadened excitation spectrum reaches into the emission spectrum. Moreover, it is described in the prior art that GQDs have no blinking properties, i.e. are not suitable for SMLM, such as by Thakur et al., "Milk-derived multi-fluorescent graphene quantum dot-based cancer theranostic system", Mater. Sci. Eng. C, 2016, volume 67, pages 468 to 477, by Zheng et al., "Glowing graphene quantum dots and carbon dots: Properties, syntheses, and biological applications", Small, 2015, volume 11, pages 1620 to 1636, by Sun et al., "Recent advances in graphene quantum dots for sensing", Mater. Today, 2013, volume 16, pages 433 to 442, and by Zhao et al., "Single photon emission from graphene quantum dots at room temperature", Nature Communications, 2018, article number 3470. Semiconductor quantum dots, such as ZnS-coated CdSe-QDs, as they are disclosed by Yang et al., "Versatile application of fluorescent quantum dot labels in super-resolution fluorescence microscopy", ACS Photonics, 2016, volume 3, pages 1611 to 1618, have comparable bad blinking properties and have a comparable broad excitation spectrum in the UV range. In addition, they are toxic. Thus, they are not suitable for performing high quality SMLM.

STED is a microscopy technique, which uses two laser beams of different wavelength. While the first laser beam is focused into the sample and excites the fluorophores contained therein so that they emit fluorescence radiation at a longer wavelength than the excitation wavelength, the second laser beam, which is called STED laser, is irradiated in form of a circular ring concentrically around the first laser beam so as to quench fluorescence in the outer peripheral area of the first laser beam. On account of this, fluorescence can only emit from the central region of a fluorophore into which the first laser beam irradiates so that the area of the sample which actually emits fluorescence is significantly smaller than the area, which is irradiated with the first excitation laser. Thus, a very sharp fluorescence emission is obtained for each fluorophore. In order to obtain a complete picture of the sample, the sample is scanned by the two lasers point to point. An important requirement for fluorophores used in STED microscopy is that they have comparable narrow excitation and emission spectra. However, as set out above the known CDs and GQDs have - on account of being mixtures with a high structural heterogeneity - comparable broad excitation and emission spectra and particularly the broad excitation spectra of GQDs reach deeply into the emission spectra. On account of this reason, both, CDs as well as GQDs limit the choice for the STED wavelength and also induce a lot of background noise. In addition, the known organic dyes are not satisfying for STED microscopy due to their comparable bad photostability leading to photobleaching.

Accordingly, the object underlying the present invention is to provide the use of a compound, which is well suitable for being used in one of and preferably in both of the high-resolution microscopy techniques STED and SMLM, which has at least when used in low concentrations good blinking properties and which is characterized by a high photon number, a very high photostability, a low toxicity, a small size around 1 nm, a comparable narrow excitation spectrum and a comparable narrow emission spectrum.

In accordance with the present invention, this object is satisfied by providing the use of a compound in single-molecule localization microscopy (SMLM) or in stimulated emission depletion microscopy (STED), wherein the compound is a substituted or unsubstituted polycyclic aromatic hydrocarbon comprising six or more substituted and/or unsubstituted aromatic hydrocarbon rings, wherein each of at least six of the six or more substituted and/or unsubstituted aromatic hydrocarbon rings is fused with at least another one of the at least six substituted and/or unsubstituted aromatic hydrocarbon rings.

This solution bases on the surprising finding that substituted or unsubstituted polycyclic aromatic hydrocarbons comprising six or more substituted and/or unsubstituted aromatic hydrocarbon rings, wherein each of at least six of the six or more substituted and/or unsubstituted aromatic hydrocarbon rings is fused with at least another one of the at least six substituted and/or unsubstituted aromatic hydrocarbon rings, has all the properties being necessary for being used in high-resolution microscopy and in particular in STED and/or SMLM. More specifically, the respective compounds have - when used in low concentrations of about 10⁻⁹ to 10⁻¹¹ molar - excellent blinking properties which are environment-independent, so that they are particularly suitable for being used in SMLM. However, in higher concentration, such as 10⁻⁵ to 10⁻⁷ molar, the compounds are heavily aggregated and stay long time in stable fluorescence mode before they enter into the blinking status. Therefore, these compounds are also useable in STED. In addition, they have a high photon number, a very high photostability, a low toxicity, a small size of 1 nm, and a comparable narrow emission spectrum. More specifically, the photon number is about 3000 to 10,000 and the duty cycle is as low as about 0.001. These compounds are further characterized by a quantum yield of up to 0.79 and by an extinction coefficient of about 70,000 M⁻¹ cm⁻¹. A particular further advantage in comparison to CDs and GQDs is that the respective compounds may be synthesized with a "bottom-up" method from smaller molecules so that compounds being homogenous both in size and structure are obtained, and not, like CDs and GQDs, a mixture of different species. This is a decisive reason why the excitation spectra as well as the emission spectra of these compounds are comparably narrow.

The term polycyclic aromatic hydrocarbon denotes in accordance with the present invention an organic molecule, which comprises at least two aromatic rings, i.e. at least two cyclic and aromatic rings, in which electrons are delocalized. Hydrocarbon means in this connection an organic molecule, which consists entirely of carbon and hydrogen atoms. Thus, an unsubstituted polycyclic aromatic hydrocarbon means in accordance with the present invention a molecule which consists entirely of carbon and hydrogen atoms, whereas a substituted polycyclic aromatic hydrocarbon is a molecule, in which one or more up to all hydrogen atoms of an unsubstituted polycyclic aromatic hydrocarbon molecule are replaced by any other atom and/or organic group and/or in which one or more of the carbon atoms are replaced by a heteroatom, such as nitrogen, oxygen, phosphorous, sulphur, boron or the like. Likewise to this, an unsubstituted aromatic hydrocarbon ring means an aromatic hydrocarbon ring which consists entirely of carbon and hydrogen atoms, whereas a substituted aromatic hydrocarbon ring is an aromatic hydrocarbon ring, in which one or more up to all hydrogen atoms of an unsubstituted aromatic hydrocarbon ring are replaced by any other atom and/or organic group and/or in which one or more of the carbon atoms are replaced by a heteroatom.

On account of its aforementioned properties, the compound is used in accordance with the present invention preferably in the high-resolution microscopy as fluorescent marker.

The use in accordance with the present invention is not particularly limited concerning the kind of high-resolution microscopy, in which the compound is used, as long as the high-resolution microscopy is one of SMLM and STED. Good results are particularly obtained, if the compound is used in any of the microscopy techniques selected from the group consisting of photoactivated localization microscopy (PALM), stochastic optical reconstruction microscopy (STORM), ground state depletion individual molecule return (GSDIM), binding activated localization microscopy (BALM) and fluorescence photo-activation localization microscopy (FPALM).

In accordance with the present invention the used compound is a substituted or unsubstituted polycyclic aromatic hydrocarbon comprising six or more substituted and/or unsubstituted aromatic hydrocarbon rings, wherein each of at least six of the six or more substituted and/or unsubstituted aromatic hydrocarbon rings is fused with at least another one of the at least six substituted and/or unsubstituted aromatic hydrocarbon rings. This means, if the compound comprises six aromatic hydrocarbon rings that each of the six aromatic hydrocarbon rings is fused with at least another one of the six aromatic hydrocarbon rings, i.e. that each of the six aromatic hydrocarbon rings shares with at least another one of the six aromatic hydrocarbon rings two neighboring carbon atoms. However, if the compound comprises more than six aromatic hydrocarbon rings, such as ten aromatic hydrocarbon rings, then each of at least six of the ten aromatic hydrocarbon rings, such as of seven of the ten aromatic hydrocarbon rings is fused with at least another one of the seven aromatic hydrocarbon rings, whereas the remaining three hydrocarbon rings are not fused with any other aromatic hydrocarbon ring. Consequently, if the compound comprises more than six aromatic hydrocarbon rings, each of the aromatic hydrocarbon rings may be fused with at least another one or only some (namely six to all but one) of the aromatic hydrocarbon rings may be fused with at least another one. All in all, the compound used in accordance with the present invention comprises a unit of at least six aromatic hydrocarbon rings, which are fused with each other.

Preferably, the compound to be used in the present invention comprises six to 91 substituted and/or unsubstituted aromatic hydrocarbon rings, wherein each of at least six of the six to 91 substituted and/or unsubstituted aromatic hydrocarbon rings is fused with at least another one of the at least six substituted and/or unsubstituted aromatic hydrocarbon rings. Thus, either each of all of the six to 91 substituted and/or unsubstituted aromatic hydrocarbon rings are fused with at least another one of the at least six substituted and/or unsubstituted aromatic hydrocarbon rings, or six to less than all of the six to 91 substituted and/or unsubstituted aromatic hydrocarbon rings are fused with at least another one of the at least six substituted and/or unsubstituted aromatic hydrocarbon rings.

In a further development of the present invention, it is proposed that the compound used in accordance with the present invention is an organic compound comprising any of the below units:
- -Ar₁(Ar₂)ₓ-, wherein the residues Ar₁ and Ar₂ are the same or different and independently from each other a substituted and/or unsubstituted aromatic hydrocarbon ring and x is in an integer of 5, 6 (only possible in the case that An is at least a C₆-ring) or 7 (only possible in the case that Ar₁ is at least a C₇-ring), i.e. -Ar₁(Ar₂)ₓ-is an unit, in which an aromatic hydrocarbon ring Ar₁ is fused with x aromatic hydrocarbon rings Ar₂,
- -An(Ar₂)ₓ-Ar₃(Ar₄)_{y}-, wherein the residues Ar₁ to Ar₄ are the same or different and independently from each other a substituted and/or unsubstituted aromatic hydrocarbon ring and x and y are independently from each other integers of 1 to 5 or 6 (only possible in the case that Ar₁/ Ar₃ is at least a C₆-ring) or 7 (only possible in the case that Ar₁/Ar₃ is at least a C₇-ring), wherein the sum of x and y is at least 4, or
- -Ari(Ar₂)ₓ-Ar₃(Ar₄)_{y}-Ar₅(Ar₆)_{z}-, wherein the residues Ar₁ to Ar₆ are the same or different and independently from each other a substituted and/or unsubstituted aromatic hydrocarbon ring and x, y and z are independently from each other integers of 1 to 5 or 6 (only possible in the case that Ar₁/Ar₃/Ars is at least a C₆-ring) or 7 (only possible in the case that Ar₁/Ar₃/Ar₅ is at least a C₇-ring), the sum of x, y and z is at least 3, or
- -Ar₁-(Ar₂)ₙ-Ar₃-, wherein the residues Ar₁ to Ar₃ are the same or different and independently from each other a substituted and/or unsubstituted aromatic hydrocarbon ring and n is an integer of 4 to 14, wherein all aromatic hydrocarbon rings may form a ring, i.e. Ar₁ and Ar₃ in the formula may be bonded or even fused with each other.

The present invention is not particularly limited concerning the type of aromatic hydrocarbon rings. Good results are particularly obtained with five-membered, six-membered and/or seven-membered aromatic rings, which may be each substituted and/or unsubstituted. Suitable examples for aromatic hydrocarbon rings of the compound to be used in the present invention are those selected from the group consisting of unsubstituted pyrrole rings, unsubstituted furan rings, unsubstituted thiophene rings, unsubstituted imidazole rings, unsubstituted pyrazole rings, unsubstituted oxazole rings, unsubstituted isoxazole rings, unsubstituted thiazole rings, unsubstituted isothiazole rings, unsubstituted benzene rings, unsubstituted pyridine rings, unsubstituted triazine rings, unsubstituted thiophene rings, unsubstituted azepine rings, unsubstituted oxepine rings, unsubstituted thiepine rings, substituted pyrrole rings, substituted furan rings, substituted thiophene rings, substituted imidazole rings, substituted pyrazole rings, substituted oxazole rings, substituted isoxazole rings, substituted thiazole rings, substituted isothiazole rings, substituted benzene rings, substituted pyridine rings, substituted triazine rings, substituted thiophene rings, substituted azepine rings, substituted oxepine rings and substituted thiepine rings.

For instance, each of substituted and/or unsubstituted aromatic hydrocarbon rings of the compound to be used may be a substituted benzene ring or an unsubstituted benzene ring. Preferably, the compound to be used in accordance with the present invention consists of six to 91 substituted and/or unsubstituted aromatic hydrocarbon rings, wherein each of the six to 91 substituted and/or unsubstituted aromatic hydrocarbon rings is fused with at least another one of the six to 91 substituted and/or unsubstituted aromatic hydrocarbon rings, and wherein each of the outer carbon atoms, i.e. of the carbon atoms with a free valency, may be substituted or unsubstituted.

Exemplary polycyclic aromatic hydrocarbons of this embodiment are:

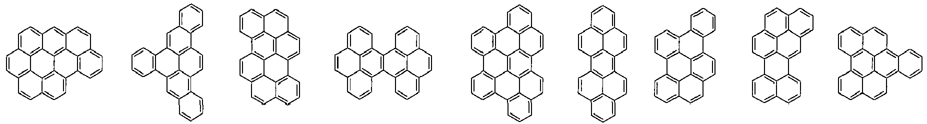

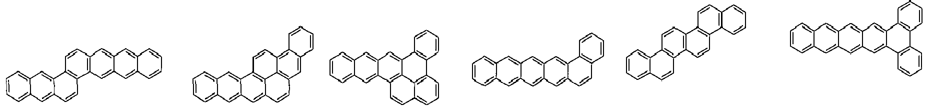

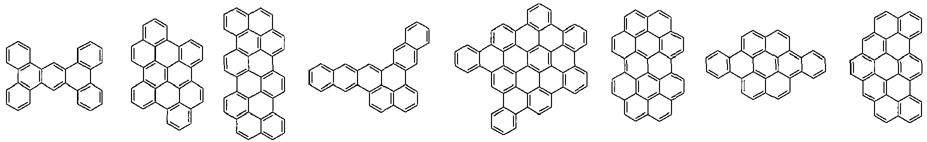

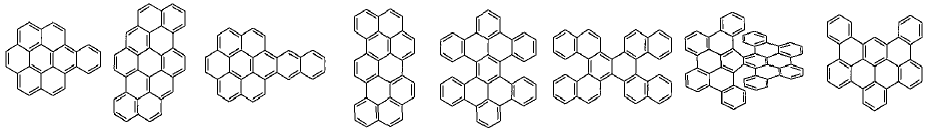

All of the rings of the above mentioned compounds are benzene rings. Any of the hydrogen atoms of the aforementioned formulae may be substituted by a substituent, such as a C₁₋₂₀-alkyl group, a C₁₋₂₀-alkenyl group, a C₁₋₂₀-alkynyl residue, C₁₋₂₀-alkoxy group, halogen atom, a C₄₋₂₀-cycloalkyl group, a C₄₋₂₀-heterocyclic group and particularly heteroaryl group, a nitro group, an amino group and/or a cyanoe group. Specific examples are decylphenyl, methoxyphenyl and nitrophenyl. The syntheses of these compounds are described by Klockenkämper et al., "Chemical Analysis: A Series of Monographs on Analytical Chemistry and Its Applications", Wiley-Interscience, August 14, 2000, by Dötz et al., "Synthesis of Large Polycyclic Aromatic Hydrocarbons: Variation of Size and Periphery", J. Am. Chem. Soc., 2000, volume 122, pages 7707 to 7717, by Kübel et al., "Synthesis and crystal packing of large polycyclic aromatic hydrocarbons: hexabenzo[bc,ef,hi,kl,no,qr]coronene and dibenzo[fg,ij]phenanthro[9,10,1,2,3-pqrst]pentaphene", J. Mater. Chem. 2000, volume 10, pages 879 to 886 and by Li et al., "Recent Progress in Chemistry of Multiple Helicenes", Chem. Asian J., 2018, volume 13, pages 884 to 894 and as described in the references cited in the aforementioned documents.

Good results are in particular obtained, when the compound to be used comprises a unit with the general formula (1) or the compound has the general formula (1): 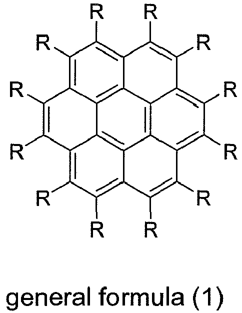 wherein in general formula (1) the residues R are the same or different and independently from each other a hydrogen atom, an unsubstituted C₁₋₂₀ hydrocarbon residue, a substitued C₁₋₂₀ hydrocarbon residue, a halogen, an azide group, a hydroxy group, a nitro group, an amino group, a formyl group, a cyano group or one or more of two adjacent residues R are linked with each other to form an aromatic group, a cycloaliphatic group or a heterocyclic goup. Any of the unsubstituted and/or substitued C₁₋₂₀ hydrocarbon residues may be an unsubstituted alkyl group, a substituted alkyl group, an unsubstituted alkenyl group, a substituted alkenyl group, an unsubstituted alkynyl group, a substituted alkynyl group, an unsubstituted alkoxy group, a substituted alkoxy group, an unsubstituted cycloalkyl group, a substituted cycloalkyl group, an unsubstituted aryl group, a substituted aryl group, an unsubstituted aralkyl group, a substituted aralkyl group, an unsubstituted hetaryl group, a substituted hetaryl group, a carboxylic acid group, a carboxylic ester group, an (alkyl or aryl)silyl group or a group formed in that two of adjacent residues of R₁ to R₈ and Ar are linked with each other to form an aromatic, heteroaromatic, cyclic or heterocyclic group. In particular, any of the unsubstituted and/or substitued C₁₋₂₀ hydrocarbon residues may be a C₁₋₂₀-alkyl group, C₁₋₂₀-alkoxy group, a C₁₋₂₀-alkenyl group, a C₁₋₂₀-alkynyl group, a C₄₋₂₀-cycloalkyl group, a C₅₋₂₀-aromatic group, a C₄₋₂₀-cycloaliphatic group or a C₅₋₂₀-heterocyclic goup. One or more of two adjacent residues R may be in particular linked with each other to form a C₅₋₂₀-aromatic group, a C₄₋₂₀-cycloaliphatic group or a C₅₋₂₀-heterocyclic goup and preferably a C₅₋₇-aromatic group, a C₆₋₈₀-cycloaliphatic group or a C₅₋₇-heterocyclic goup.

In accordance with a particularly preferred embodiment of the present invention, the compound to be used comprises a unit with the general formula (2) or the compound to be used has the general formula (2): 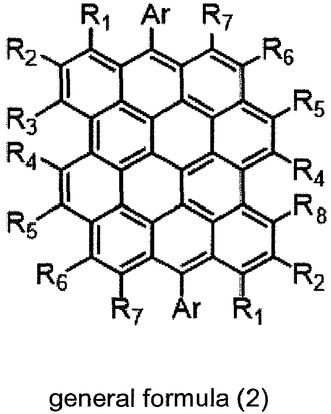 wherein in general formula (2):
residues R₁ to R₈ and Ar are independently from each other selected from the group consisting of hydrogen, unsubstituted alkyl groups, substituted alkyl groups, unsubstituted alkenyl groups, substituted alkenyl groups, unsubstituted alkynyl groups, substituted alkynyl groups, unsubstituted alkoxy groups, substituted alkoxy groups, unsubstituted cycloalkyl groups, substituted cycloalkyl groups, unsubstituted aryl groups, substituted aryl groups, unsubstituted aralkyl groups, substituted aralkyl groups, unsubstituted hetaryl groups, substituted hetaryl groups, azide groups, hydroxy groups, nitro groups, amino groups, formyl groups, carboxylic acid groups, carboxylic ester groups, cyano groups, (alkyl or aryl)silyl groups and groups formed in that two of adjacent residues of R₁ to R₈ and Ar are linked with each other to form an aromatic, heteroaromatic, cyclic or heterocyclic group. The skeleton of this formula, i.e. the fused benzene residues or aromatic core structure, respectively, without the residues R₁ to R₈ and Ar, is subsequently also referred to as DBOV as abbreviation for dibenzo[hi,st]ovalene.

Each of both residues R₁ are the same, each of both residues R₂ are the same, each of both residues R₃ are the same, each of both residues R₄ are the same, each of both residues R₅ are the same, each of both residues R₆ are the same and each of both residues R₇ are the same, whereas residues R₁ may be the same or different to the other residues, residues R₂ may be the same or different to the other residues and so forth.

Preferably, the residues R in the general formula (1) and in particular the residues R₁ to R₈ and Ar in the general formula (2) are independently from each other selected from the group consisting of hydrogen; unsubstituted linear or branched C₁₋₃₀-alkyl groups; substituted linear or branched C₁₋₃₀-alkyl groups whose hydrocarbon chain is interrupted by one or more -O-, -S-, -NR'- with R' being C₁₋₃₀-alkyl, C₁₋₃₀-alkenyl, C₁₋₃₀-alkynyl, C₁₋₃₀-alkoxy, C₆₋₂₀-aryl, C₆₋₂₀-heteroaryl, -CO- and/or -SO- groups; linear or branched C₁₋₃₀-alkyl groups whose hydrocarbon chain is monosubstituted or polysubstituted with a carboxyl group, a sulfo group, a hydroxyl group, a cyano group, a C-C-alkoxy group and/or with a 5- to 7-membered heterocyclic group which is bonded via a nitrogen atom to the hydrocarbon chain; trialkylsilyl alkynyl groups; unsubstituted C₃₋₃₀-cycloalkyl groups; substituted C₁₋₃₀-cycloalkyl groups whose hydrocarbon chain is interrupted by one or more -O-, -S-, -NR'- with R' being C₁₋₃₀-alkyl, C₁₋₃₀-alkenyl, C₁₋₃₀-alkynyl, C₁₋₃₀-alkoxy, C₆₋₂₀-aryl, C₆₋₂₀-heteroaryl, -CO- and/or -SO- groups; linear or branched C₁₋₃₀-cycloalkyl groups whose hydrocarbon chain is monosubstituted or polysubstituted with a carboxyl group, a sulfo group, a hydroxyl group, a cyano group, a C-C-alkoxy group and/or with a 5- to 7-membered heterocyclic group which is bonded via a nitrogen atom to the hydrocarbon chain; phenyl groups; naphthyl groups; anthryl groups; pyrenyl groups; phenyl-, naphthyl-, anthryl- or pyrenyl-groups being monosubstituted or polysubstituted with C₁₋₁₈-alkyl, C₁₋₁₈-alkoxy, halogen, hydroxyl, cyano, carboxyl, -CONHR with R being C₁₋₃₀-alkyl, C₁₋₃₀-alkenyl, C₁₋₃₀-alkynyl, C₁₋₃₀-alkoxy, C₆₋₂₀-aryl or C₆₋₂₀-heteroaryl, -NHCOR with R being C₁₋₃₀-alkyl, C₁₋₃₀-alkenyl, C₁₋₃₀-alkynyl, C₁₋₃₀-alkoxy, C₆₋₂₀-aryl, C₆₋₂₀-heteroaryl; azide groups and groups formed in that two of adjacent residues of R₁ to R₈ and Ar are linked with each other to form an aromatic, heteroaromatic, cyclic or heterocyclic group.

More preferably, the residues R in the general formula (1) and in particular the residues R₁ to R₈ and Ar in the general formula (2) are independently from each other selected from the group consisting of hydrogen, unsubstituted linear or branched C₁₋₃₀-alkyl groups, unsubstituted linear or branched C₁₋₃₀-alkoxy groups, unsubstituted C₃₋₃₀-cycloalkyl groups, phenyl groups, naphthyl groups, anthryl groups, pyrenyl groups, azide groups, polyethylene groups with 2 to 20 ethylene moieties, phenyl ethylene, triisopropylsilyl ethynyl, trimethylsilyl ethynyl, and groups formed in that two of adjacent residues of R₁ to R₈ and Ar are linked with each other to form an aromatic, heteroaromatic, cyclic or heterocyclic group.

Specific examples for the residues R in the general formula (1) and in particular the residues R₁ to R₈ and Ar in the general formula (2) are:
Hydrogen, methyl, ethyl, propyl, isopropyl, butyl, isobutyl, tert-butyl, pentyl, isopentyl, neopentyl, *tert*-pentyl, hexyl, 2-methylpentyl, heptyl, 1-ethylpentyl, octyl, 2-ethylhexyl, isooctyl, nonyl, isononyl, decyl, isodecyl, undecyl, dodecyl, tridecyl, isotridecyl, tetradecyl, pentadecyl, hexadecyl, heptadecyl, octadecyl, nonadecyl and eicosyl (the above names isooctyl, isononyl, isodecyl and isotridecyl are trivial names and originate from the alcohols obtained by the oxo synthesis); 2-methoxyethyl, 2-ethoxyethyl, 2-propoxyethyl, 2-isopropoxyethyl, 2-butoxyethyl, 2- and 3-methoxypropyl, 2- and 3-ethoxypropyl, 2- and 3-propoxypropyl, 2- and 3-butoxypropyl, 2- and 4-methoxybutyl, 2- and 4-ethoxybutyl, 2- and 4-propoxybutyl, 3,6-dioxaheptyl, 3,6-dioxaoctyl, 4,8-dioxanonyl, 3,7-dioxaoctyl, 3,7-dioxanonyl, 4,7-dioxaoctyl, 4,7-dioxanonyl, 2- and 4-butoxybutyl, 4,8-dioxadecyl, 3,6,9-trioxadecyl, 3,6,9-trioxaundecyl, 3,6,9-trioxadodecyl, 3,6,9,12-tetraoxatridecyl and 3,6,9,12-tetraoxatetradecyl; 2-methylthioethyl, 2-ethylthioethyl 2-propylthioethyl, 2-isopropylthioethyl, 2-butylthioethyl, 2- and 3-methylthiopropyl, 2- and 3-ethylthiopropyl, 2- and 3-propylthiopropyl, 2- and 3-butylthiopropyl, 2- and 4-methylthiobutyl, 2- and 4-ethylthiobutyl, 2- and 4-propylthiobutyl, 3,6-dithiaheptyl, 3,6-dithiaoctyl, 4,8-dithianonyl, 3,7-dithiaoctyl, 3,7-dithianonyl, 2- and 4-butylthiobutyl, 4,8-dithiadecyl, 3,6,9-trithiadecyl, 3,6,9-trithiaundecyl, 3,6,9-trithiadodecyl, 3,6,9,12-tetrathiatridecyl and 3,6,9,12-tetrathiatetradecyl; 2-monomethyl- and 2-monoethylaminoethyl, 2-dimethylaminoethyl, 2- and 3-dimethylaminopropyl, 3-monoisopropylaminopropyl, 2- and 4-monopropylaminobutyl, 2 and 4-dimethylaminobutyl, 6-methyl-3,6-diazaheptyl, 3,6-dimethyl-3,6-diazaheptyl, 3,6-diazaoctyl, 3,6-dimethyl-3,6-diazaoctyl, 9-methyl-3,6,9-triazadecyl, 3,6,9-trimethyl-3,6,9-triazadecyl, 3,6,9-triazaundecyl, 3,6,9-trimethyl-3,6,9-triazaundecyl, 12-methyl-3,6,9,12-tetraazatridecyl and 3,6,9,12-tetramethyl-3,6,9,12-tetraazatridecyl; propan-2-on-1-yl, butan-3-on-1-yl, butan-3-on-2-yl and 2-ethylpentan-3-on-1-yl; 2-methylsulfonylethyl, 2-ethylsulfonylethyl 2-propylsulfonylethyl, 2-isopropylsulfonylethyl, 2-butylsulfonylethyl, 2- and 3-methylsulfonylpropyl, 2- and 3-ethylsulfonylpropyl, 2- and 3-propylsulfonylpropyl, 2- and 3-butylsulfonylpropyl, 2- and 4-methylsulfonylbutyl, 2- and 4-ethylsulfonylbutyl, 2- and 4-propylsulfonylbutyl and 4-butylsulfonylbutyl; carboxymethyl, 2-carboxyethyl, 3-carboxypropyl, 4-carboxybutyl, 5-carboxypentyl, 6-carboxyhexyl, 8-carboxyoctyl, 10-carboxydecyl, 12-carboxydodecyl and 14-carboxytetradecyl;
sulfomethyl, 2-sulfoethyl, 3-sulfopropyl, 4-sulfobutyl, 5-sulfopentyl, 6-sulfohexyl, 8-sulfooctyl, 10-sulfodecyl, 12-sulfododecyl and 14-sulfotetradecyl; 2-hydroxyethyl, 3-hydroxypropyl, 1-hydroxyprop-2-yl 2- and 4-hydroxybutyl, 1-hydroxybut-2-yl and 8-hydroxy-4-oxaoctyl; cyanomethyl 2-cyanoethyl, 3-cyanopropyl, 2-methyl-3-ethyl-3-cyanopropyl, 7-cyano-7-ethylheptyl and 4,7-dimethyl-7-cyanoheptyl;
methoxy, ethoxy, propoxy, isopropoxy, butoxy, isobutoxy, sec-butoxy, *tert-*butoxy, pentoxy, isopentoxy, neopentoxy, *tert-*pentoxy and hexoxy; carbamoyl, methylaminocarbonyl, ethylaminocarbonyl, propylaminocarbonyl, butylaminocarbonyl, pentylaminocarbonyl, hexylaminocarbonyl, heptylaminocarbonyl, octylaminocarbonyl, nonylaminocarbonyl, decylaminocarbonyl and phenylaminocarbonyl; formylamino, acetylamino, propionylamino and benzoylamino; chlorine, bromine and iodine; phenylazo, 2-naphthylazo, 2-pyridylazo and 2-pyrimidylazo; phenyl, 2-naphthyl, 2- and 3-pyrryl, 2-, 3- and 4-pyridyl, 2-,4- and 5-pyrimidyl, 3-, 4- and 5-pyrazolyl, 2-, 4- and 5-imidazolyl, 2-, 4- and 5-thiazolyl, 3-(1,2,4-triazyl), 2-(1,3,5-triazyl), 6-quinaldyl, 3-, 5-, 6- and 8-quinolinyl, 2-benzoxazolyl, 2-benzothiazolyl, 5-benzothiadiazolyl, 2- and 5-benzimidazolyl and 1- and 5-isoquinolyl; 2-, 3- and 4-methylphenyl, 2,4-, 2,5-, 3,5- and 2,6-dimethylphenyl, 2,4,6-trimethylphenyl, 2-, 3- and 4-ethylphenyl, 2,4-, 2,5-, 3,5- and 2,6-diethylphenyl, 2,4,6-triethylphenyl, 2-, 3- and 4-propylphenyl, 2,4-, 2,5-, 3,5- and 2,6-dipropylphenyl, 2,4,6-tripropylphenyl, 2-, 3- and 4-isopropylphenyl, 2,4-, 2,5-, 3,5- and 2,6-diisopropylphenyl, 2,4,6-triisopropylphenyl, 2-, 3- and 4-butylphenyl, 2,4-, 2,5-, 3,5- and 2,6-dibutylphenyl, 2,4,6-tributylphenyl, 2-, 3- and 4-isobutylphenyl, 2,4-, 2,5-, 3,5- and 2,6-diisobutylphenyl, 2,4,6-triisobutylphenyl, 2-, 3- and 4-sec-butylphenyl, 2,4-, 2,5-, 3,5- and 2,6-di-sec-butylphenyl and 2,4,6-tri-sec-butylphenyl, 2-, 3- and 4-*tert*-butylphenyl, 2,4-, 2,5-, 3,5- and 2,6-di-*tert-*butylphenyl, 2,4,6-tri-tertbutylphenyl; 2-, 3- and 4-methoxyphenyl, 2,4-, 2,5-, 3,5- and 2,6-dimethoxyphenyl, 2,4,6-trimethoxyphenyl, 2-, 3- and 4-ethoxyphenyl, 2,4-, 2,5-, 3,5- and 2,6-diethoxyphenyl, 2,4,6-triethoxyphenyl, 2-, 3- and 4-propoxyphenyl, 2,4-, 2,5-, 3,5- and 2,6-dipropoxyphenyl, 2-, 3- and 4-isopropoxyphenyl, 2,4-, 2,5-, 3,5- and 2,6-diisopropoxyphenyl and 2-, 3- and 4-butoxyphenyl; 2-, 3- and 4-chlorophenyl, and 2,4-, 2,5-, 3,5- and 2,6-dichlorophenyl; 2-, 3- and 4-hydroxyphenyl and 2,4-, 2,5-, 3,5- and 2,6-dihydroxyphenyl; 2-, 3- and 4-cyanophenyl; 3- and 4-carboxyphenyl; 3- and 4-carboxamidophenyl, 3- and 4-N-methylcarboxamidophenyl and 3- and 4-N-ethylcarboxamidophenyl, 3- and 4-acetylaminophenyl, 3- and 4-propionylaminophenyl and 3- and 4-butyrylaminophenyl; 3- and 4-N-phenylaminophenyl, 3- and 4-N-(o-tolyl)aminophenyl, 3- and 4-N-(m-tolyl)aminophenyl and 3- and 4-N-(p-tolyl)aminophenyl, 3- and 4-(2-pyridyl)aminophenyl, 3- and 4-(3-pyridyl)aminophenyl, 3- and 4-(4-pyridyl)aminophenyl, 3- and 4-(2-pyrimidyl)aminophenyl and 4-(4-pyrimidyl)aminophenyl; 4-phenylazophenyl, 4-(1-naphthylazo)phenyl, 4-(2-naphthylazo)phenyl, 4-(4-naphthylazo)phenyl, 4-(2-pyridylazo)phenyl, 4-(3-pyridylazo)phenyl, 4-(4-pyridylazo)phenyl, 4-(2-pyrimidylazo)phenyl, 4-(4-pyrimidylazo)phenyl and 4-(5-pyrimidylazo)phenyl; cyclopentyl, 2- and 3-methylcyclopentyl, 2- and 3-ethylcyclopentyl, cyclohexyl, 2-, 3- and 4-methylcyclohexyl, 2-, 3- and 4-ethylcyclohexyl, 3- and 4-propylcyclohexyl, 3- and 4-isopropylcyclohexyl, 3- and 4-butylcyclohexyl, 3- and 4-sec-butylcyclohexyl, 3- and 4-*tert*-butylcyclohexyl, cycloheptyl, 2-, 3- and 4-methylcycloheptyl, 2-, 3- and 4-ethylcycloheptyl, 3- and 4-propylcycloheptyl, 3- and 4-isopropylcycloheptyl, 3- and 4-butylcycloheptyl, 3- and 4-sec-butylcycloheptyl, 3- and 4-*tert*-butylcycloheptyl, cyclooctyl, 2-, 3-, 4- and 5-methylcyclooctyl, 2-, 3-, 4 and 5-ethylcyclooctyl, 3-, 4- and 5-propylcyclooctyl, 2-dioxanyl, 4-morpholinyl, 2- and 3-tetrahydrofuryl, 1-, 2- and 3-pyrrolidinyl and 1-, 2-, 3- and 4-piperidyl; phenylazide, 2-naphthylazide, 2-pyridylazd; alkynyl groups, e.g., ethynyl, propynyl, butynyl, pentynyl, hexynyl, heptynyl, octynyl, nonynyl, decynyl.

Preferably, the residues R₁ to R₈ and Ar in the general formula (2) are the same or different and independently from each other a hydrogen atom, a C₁₋₂₀-alkyl group, a C₁₋₂₀-alkenyl group, a C₁₋₂₀-alkynyl group, a C₄₋₂₀-cycloalkyl group, a C₅₋₂₀-aromatic group, a C₄₋₂₀-cycloaliphatic group or a C₅₋₂₀-heterocyclic goup. One or more of two adjacent residues R may be in particular linked with each other to form a C₅-₂₀-aromatic group, a C₄₋₂₀-cycloaliphatic group or a C₅₋₂₀-heterocyclic goup and preferably a C₅₋₇-aromatic group, a C₆₋₈₀-cycloaliphatic group or a C₅₋₇-heterocyclic goup.

Particularly preferably, in general formula (2) residue R₁ is hydrogen or a C₁₋₂₀-alkyl group, residues R₂ to R₈ are hydrogen and residue Ar is aryl, a C₆₋₁₅-alkyl group or a trialkylsilyl alkynyl group.

In any of the aforementioned embodiments, the residue Ar in the general formula (2) is preferably selected from the group consisting of phenyl, trifluorphenyl, 1,5-dimethylphenyl, mesityl, triisopropylsilyl ethynyl, trimethylsilyl ethynyl, phenylsilyl ethynyl and C₆₋₁₅-alkyl groups.

In accordance with another particularly preferred embodiment of the present invention, in the general formula (2) either: i) residue R₁ is a C₁₋₂₀-alkyl group and residues R₂ to R₈ are hydrogen, or ii) residues R₁ to R₈ are hydrogen or iii) residues R₁, R₂, R₄ to R₇ are hydrogen and residues R₃ and R₈ are the same or different and mesityl or triisopropylsilyl ethynyl, wherein Ar is as described above.

Most preferably, the compound to be used in accordance with the present invention has one of the below formulae: 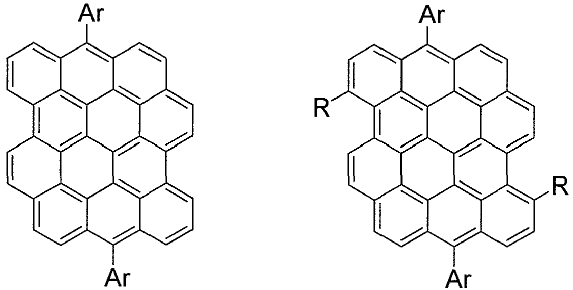 wherein: 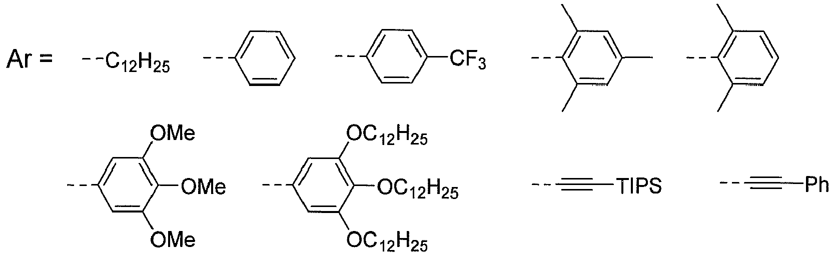 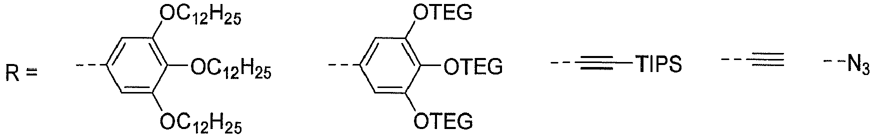 with Ph=phenyl, Me=methyl and TIPS=triisopropylsilyl. Particularly suitable examples therefore are:

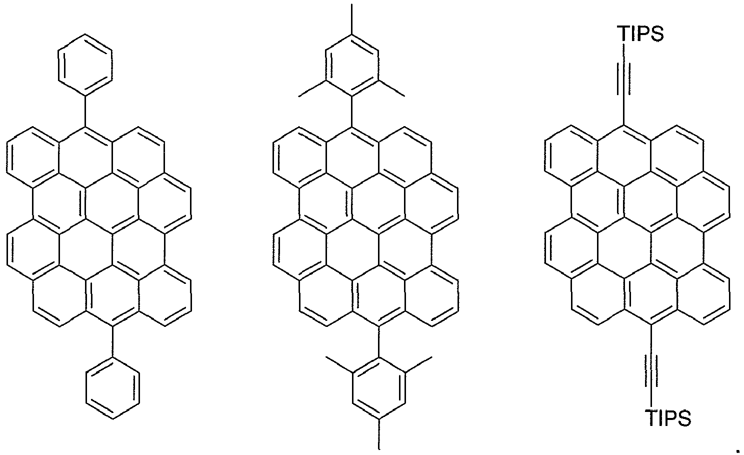

In accordance with an alternative embodiment, in the general formula (2) one or both of the residues R₃ and R₄ are linked with each other to form an aromatic, heteroaromatic, cyclic or heterocyclic group. Preferably, one or both of the residues R₃ and R₄ are linked with each other in this embodiment to form a heterocyclic group, such as in particular a heterocyclic imide group, more preferably a C₁₋₁₂-N-alkyl imide group and most preferably a N-hexyl imide group.

Likewise to this, in the general formula (2) one or both of the residues R₃ and R₄ may be linked with each other to form a fumaric acid imide group, preferably a C₁₋₁₂-N-alkyl fumaric acid imide group and more preferably a N-hexyl fumaric acid imide group. In this embodiment it is preferred that residue R₁ is hydrogen or a C₁₋₂₀-alkyl group, residues R₂ to R₈ are hydrogen and residue Ar is aryl or a trialkylsilyl alkynyl group.

Examples for compound in accordance with this embodiment are:

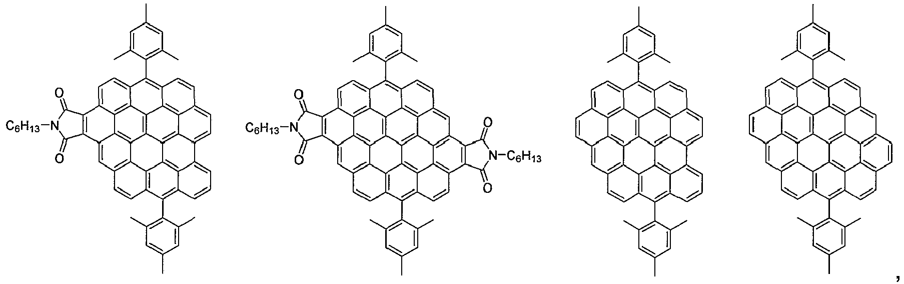

In accordance with a further particularly preferred embodiment of the present invention, the compound having the general formula (1) or the general formula (2) has a solubility in water at 23°C of at least 0.01 g/l, preferably of at least 0.05 g/l and more preferably of at least 0.1 g/l. These compounds are biocompatible and therefore excellently suitable for being used in SMLM and STED bio-imaging experiments, such as when the sample for the microscopy is a bioogical system, such as a cell.

In accordance with the present invention, the solubility and particularly the solubility of the compound having the general formula (1) or the general formula (2) in water at 23°C is measured as follows: 1 mg sample is weighed into an Erlenmeyer flask and is then mixed homogeneously with 4 ml of water with a spatula at 23°C. The remaining substance on the spatula is stripped off with a magnetic stirrer bar, which is then added to the Erlenmeyer flask. While stirring with a magnetic stirrer, water is added dropwise with a burette until the solution in the Erlenmeyer flask becomes clear at 23°C. After that, the solubility of compound in water is calculated by calculating the ratio of weighed compound divided by the volume of added water and then by recalculating this ratio to 1 I of water.

Preferably, in this embodiment in the general formula (1) at least one of residues R or in the general formula (2) at least one of residues R₁ to R₈ and Ar is a hydrophilic group selected from the group consisting of groups comprising one or more carboxy groups, groups including one or more polyethylene residues with each 2 to 20 alkylene moieties and preferably 2 to 20 ethylene moieties, one or more sulfonate groups, one or more quaternary amine groups, one or more amide groups, one or more imine groups and one or more pyridine groups.

It is further preferred that in this embodiment in the general formula (1) at least one of residues R or in the general formula (2) at least one of residues R₁ to R₈ and Ar is a hydrophilic group according to one of the below formulae:

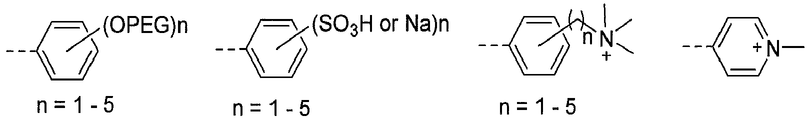

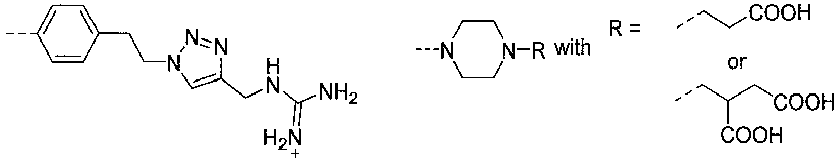

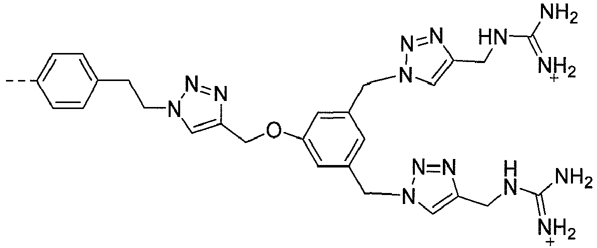

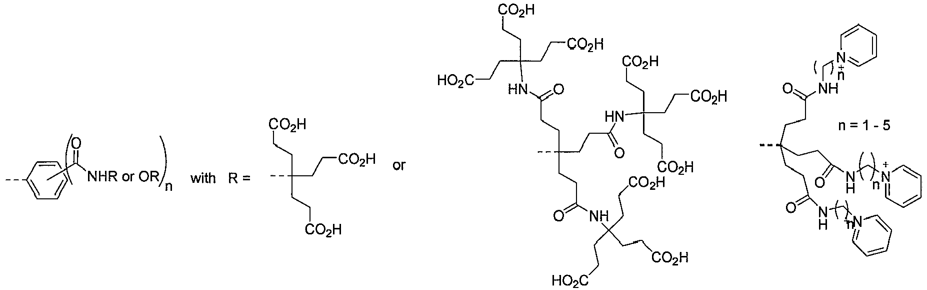

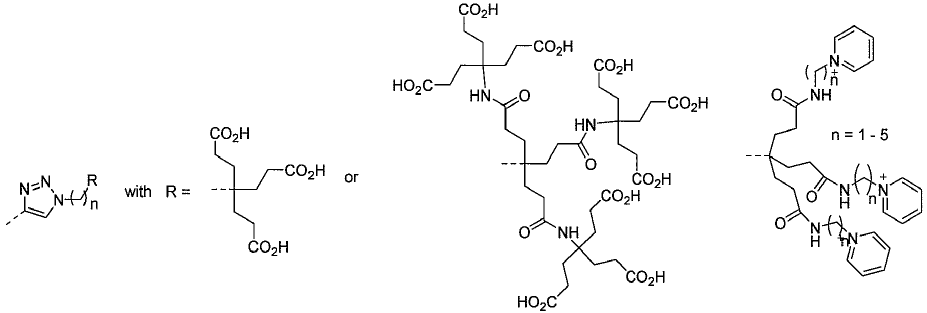

In a further development of the idea of the present invention, it is further preferred in this embodiment that in general formula (2) at least one of residues R₁ to R₈ and Ar is a group including one to five, preferably two to four and more preferably three polyethylene residues. Preferably, each of the polyethylene residues comprises 2 to 20, more preferably 2 to 8 and still more preferably 3 to 5, such as in particular 4 ethylene moieties. At least one of residues R in the general formula (1) or at least one residues R₁ to R₈ and Ar in the general formula (2) of this variant is preferably a phenyl group, which is substituted with one to five, preferably two to four and more preferably three polyethylene residues with each 2 to 20, preferably 2 to 8 and more preferably 3 to 5, such as in particular 4 ethylene moieties.

A particular good water solubility is obtained, when each of residues R₃, R₈ and Ar in the general formula (2) is a hydrophilic group, or when each of residues R₃ and R₈ is a hydrophilic group and residue Ar is selected from the group consisting of phenyl, mesitylene, triisopropylsilyl ethynyl and trimethylsilyl ethynyl. All of the hydrophilic groups of the compound may be the same or may be different. Residues R₁, R₂ and R₄ to R₇ are in this variant of the present invention preferably hydrogen.

In a further development of the idea of the present invention it is proposed that at least one of residues R in the general formula (1) and at least one of the residues R₁ to R₈ and Ar and preferably exactly one of the residues R₁ to R₈ and Ar of the general formula (2) is a group, which easily reacts with a respective coupling group at the target molecule. For instance, any of one or more of the residues R in the general formula (1) and any of one or more of the residues R₁ to R₈ and Ar of the general formula (2) is a group with a terminal alkyne group. Such groups can be easily reacted for instance with an azide group of the target molecule, wherein the alkyne group and the azide group react under formation of a triazole group, which connects the fluorophore to the target molecule. Preferably, one of the residues R₁ to R₈ and Ar of the compound having the general formula (2) is an ethyne group.

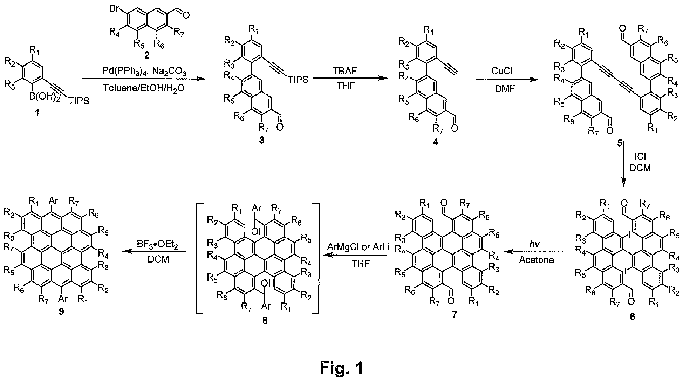

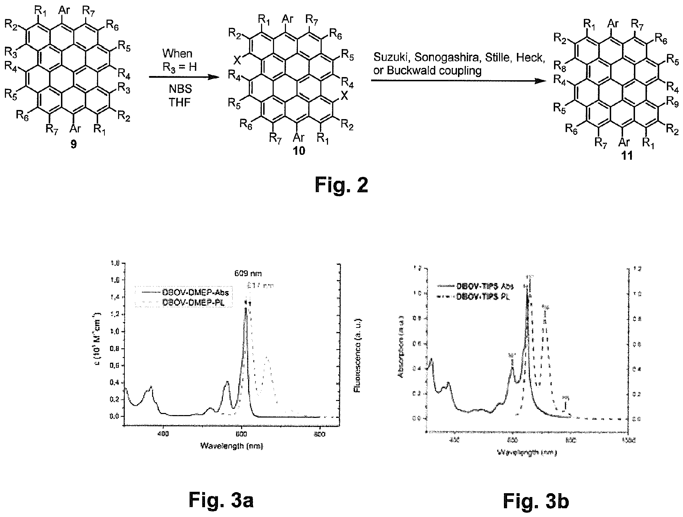

The DBOV compounds having the general formula (2) can be synthesized according to the reaction schemes shown in figures 1 and 2 and as described below in the examples.

In more general terms, it is preferred that the compound to be used in accordance with the present invention comprises a unit with the general formula (3) or the compound has the general formula (3): 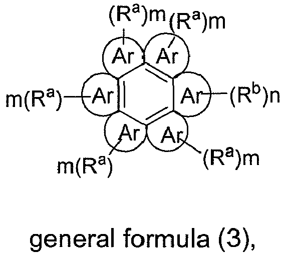 wherein in general formula (3) the residues R^{a} and R^{b} are the same or different and independently from each other a hydrogen atom, an unsubstituted C₁₋₂₀ hydrocarbon residue, a substitued C₁₋₂₀ hydrocarbon residue, a halogen, an azide group, a hydroxy group, a nitro group, an amino group, a formyl group, a cyano group or one or more of two adjacent residues R^{a} and R^{b} are linked with each other to form an aromatic group, a cycloaliphatic group or a heterocyclic goup, each of residues Ar is an aromatic group, a cycloaliphatic group or a heterocyclic goup, and m is an integer of 1 to 4. Any of the unsubstituted and/or substitued C₁₋₂₀ hydrocarbon residues may be in particular a C₁₋₂₀-alkyl group, a C₁₋₂₀-alkenyl group, a C₁₋₂₀-alkynyl group, C₁₋₂₀-alkoxy group, a C₄₋₂₀-cycloalkyl group, a C₅₋₂₀-aromatic group, a C₄₋₂₀-cycloaliphatic group or a C₅₋₂₀-heterocyclic goup. One or more of two adjacent residues R may be in particular linked with each other to form a C₅₋₂₀-aromatic group, a C₄₋₂₀-cycloaliphatic group or a C₅₋₂₀-heterocyclic goup and preferably a C₅₋₇-aromatic group, a C₆₋₈₀-cycloaliphatic group or a C₅₋₇-heterocyclic goup.

In the forementioned embodiment, at least one of the residues Ar in the general formula (3) may be a heterocyclic group and each of the remaining residues Ar may be independently from each other a benzene group, a cycloaliphatic group or a heterocyclic goup.

More preferably, the residues R^{a} and R^{b} in the general formula (3) are the same or different and independently from each other a hydrogen atom, an unsubstituted C₁₋₂₀-hydrocarbon residue or one or more of two adjacent residues R are linked with each other to form an aromatic group or a heterocyclic goup.

Suitable examples for the use of this embodiment of the present invention are compounds falling under the general formula (3), which have any of the below general formulae (4) and (5): 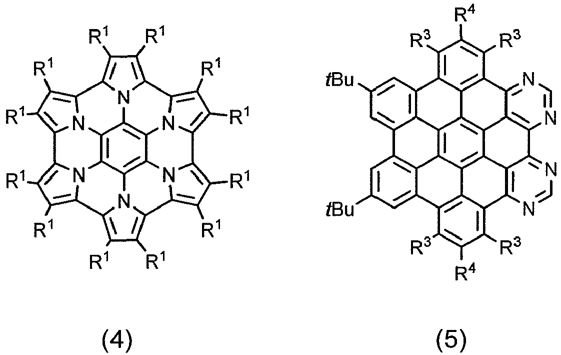 wherein in the general formulae (4) and (5) the residues R₁ to R₄ are the same or different and independently from each other a hydrogen atom, an unsubstituted C₁₋₂₀-hydrocarbon residue, a substitued C₁₋₂₀ hydrocarbon residue, a halogen, an azide group, a hydroxy group, a nitro group, an amino group, a formyl group, a cyano group or one or more of two adjacent residues R₁ to R₄ are linked with each other to form an aromatic group, a cycloaliphatic group or a heterocyclic goup, and each of residues Ar is a cyclic group, wherein at least one of the residues Ar is a heterocyclic group and each of the remaining residues Ar is independently from each other a benzene group, a cycloaliphatic group or a heterocyclic goup. Any of the unsubstituted and/or substitued C₁₋₂₀ hydrocarbon residues may be in particular a C₁₋₂₀-alkyl group, a C₁₋₂₀-alkenyl group, a C₁₋₂₀-alkynyl group, a C₁₋₂₀-alkoxy group, a C₄₋₂₀-cycloalkyl group, a C₅₋₂₀-aromatic group, a C₄₋₂₀-cycloaliphatic group or a C₅-₂₀-heterocyclic goup. One or more of two adjacent residues R may be in particular linked with each other to form a C₅₋₂₀-aromatic group, a C₄₋₂₀-cycloaliphatic group or a C₅₋₂₀-heterocyclic goup and preferably a C₅₋₇-aromatic group, a C₆₋₈₀-cycloaliphatic group or a C₅₋₇-heterocyclic goup.

The synthesis of compounds according to the general formulae (4) and (5) are described by Takase, "Annularly dfused hexapyrrolohexaazacoronenes: An extended π-system with multiple interior nitrogen atoms displays stable oxidation states", Angew. Chem. Int. Ed., 2007, volume 46, pages 5524 to 5527 and by Draper et al., "Heterosuperbenzenes: A new family of nitrogen-functionalized, graphitic molecules", J. Am. Chem. Soc., 2002, volume 124, pages 3486 to 3487.

Other suitable examples for compounds to be used in accordance with the present invention are compounds, which comprise a unit with any of the below formulae or which have any of the below formulae:

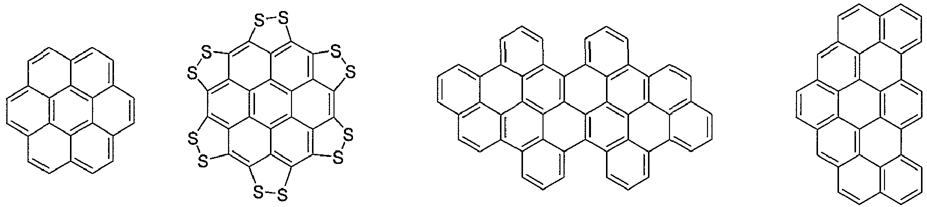

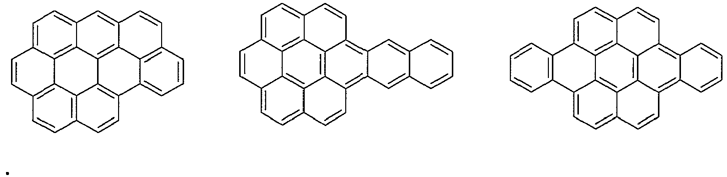

Any of the hydrogen atoms of the above shown formulae may be substituted with any of the aforementioned substituents.

Further suitable examples for the compound to be used in the present invention are the below general formulae (6) to (22): 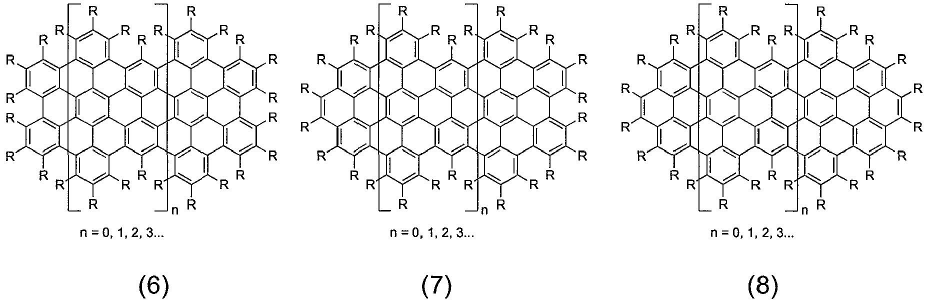 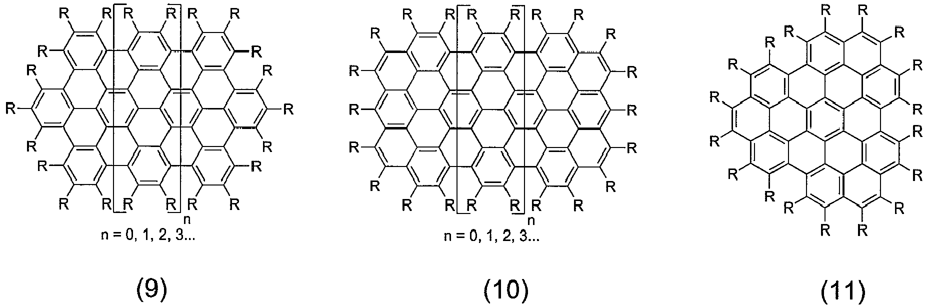 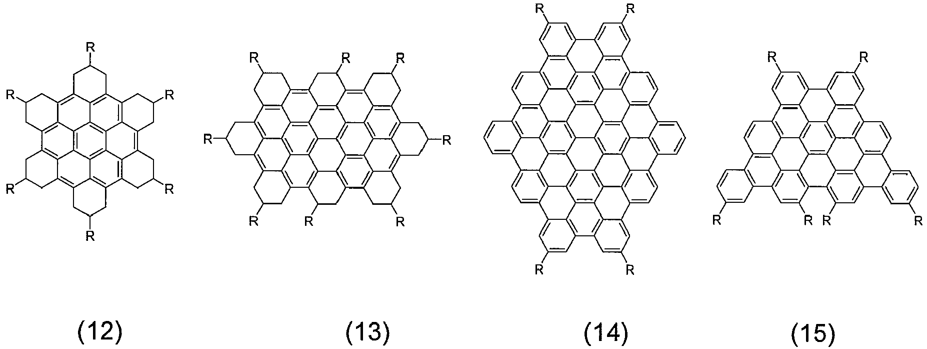 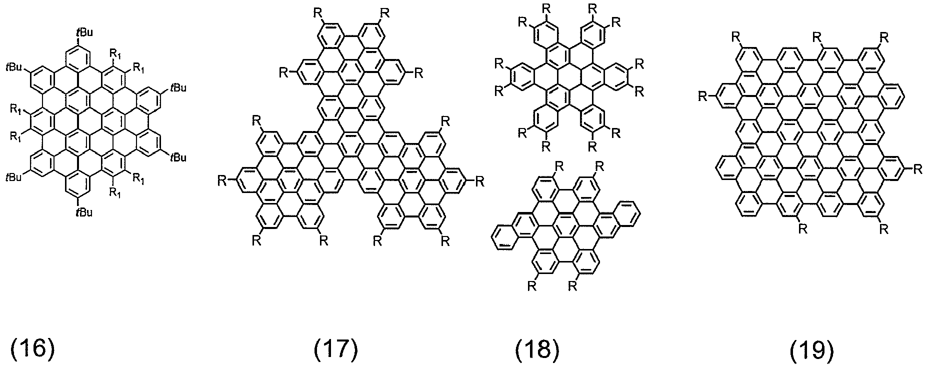 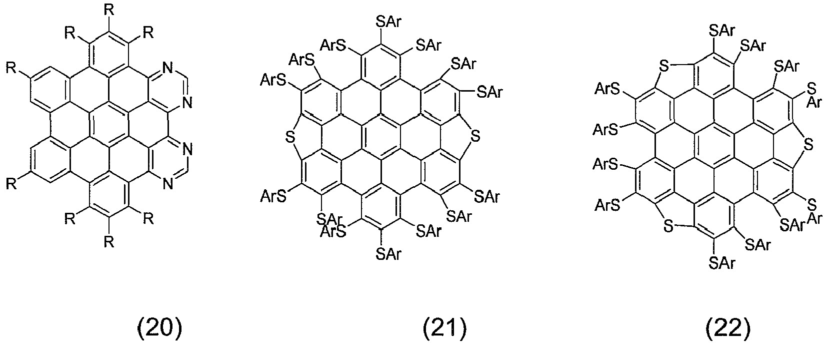 wherein in each of the general the formulae (6) to (22) the residues R, R', R₁ and Ar are the same or different and independently from each other a hydrogen atom, a C₁₋₂₀ alkyl group or a halogen, and wherein n is an integer between 1 and 10.

Further specific examples for suitable are those having any of the below formulae:

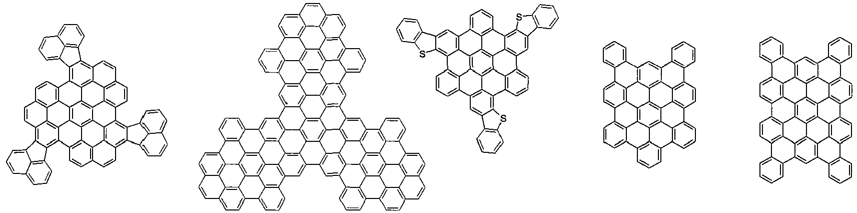

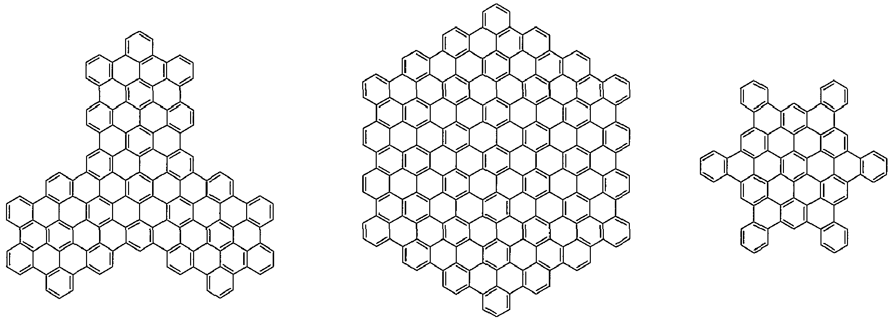

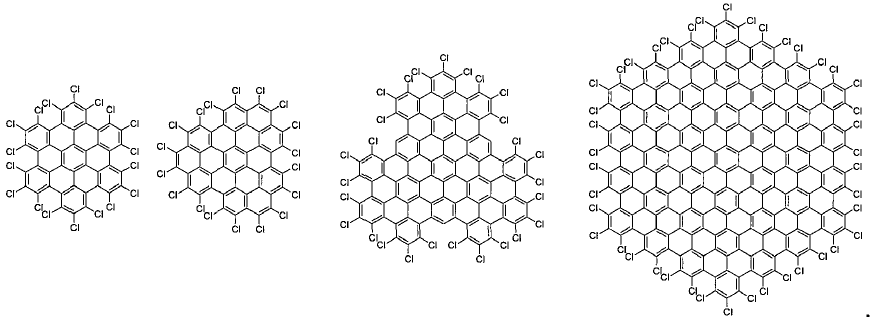

Preferred compounds are:

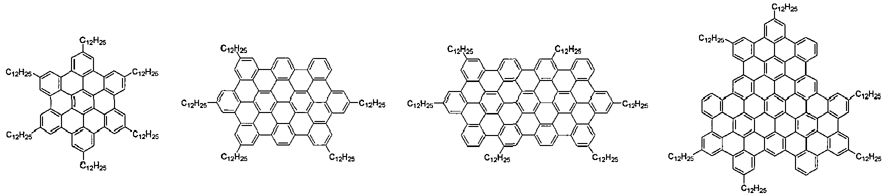

In accordance with still an alternative embodiment, the compound to be used in the present invention comprises a unit with the general formula (23) or the compound has the general formula (23): 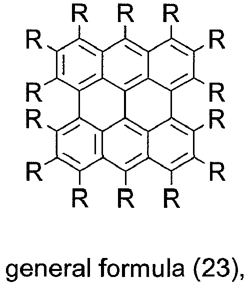 wherein in general formula (23) the residues R are the same or different and independently from each other a hydrogen atom, an unsubstituted C₁₋₂₀ hydrocarbon residue, a substitued C₁₋₂₀ hydrocarbon residue, a halogen, an azide group, a hydroxy group, a nitro group, an amino group, a formyl group, a cyano group or one or more of two adjacent residues R are linked with each other to form an aromatic group, a cycloaliphatic group or a heterocyclic goup. Any of the unsubstituted and/or substitued C₁₋₂₀ hydrocarbon residues may be in particular a C₁₋₂₀-alkyl group, a C₁₋₂₀-alkenyl group, a C₁₋₂₀-alkynyl group, a C₁₋₂₀-alkoxy group, a C₄₋₂₀-cycloalkyl group, a C₅₋₂₀-aromatic group, a C₄₋₂₀-cycloaliphatic group or a C₅-₂₀-heterocyclic goup. One or more of two adjacent residues R may be in particular linked with each other to form a C₅₋₂₀-aromatic group, a C₄₋₂₀-cycloaliphatic group or a C₅₋₂₀-heterocyclic goup and preferably a C₅₋₇-aromatic group, a C₆₋₈₀-cycloaliphatic group or a C₅₋₇-heterocyclic goup.

Preferably, each of residues R in the general formula (23) is a hydrogen atom and/or one or more of two adjacent residues R are linked with each other to form an aromatic group.

Specific examples for suitable compounds according to the general formula (23) are those having any of the below formulae:

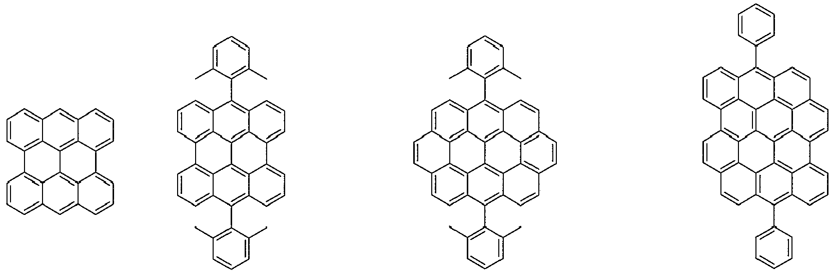

In accordance with still an alternative embodiment, the compound to be used in the present invention comprises a unit with the general formula (24) or the compound has the general formula (24): 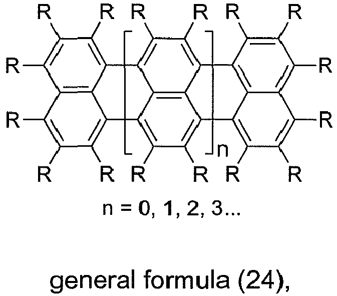 wherein in general formula (24) the residues R are the same or different and independently from each other a hydrogen atom, an unsubstituted C₁₋₂₀ hydrocarbon residue, a substitued C₁₋₂₀ hydrocarbon residue, a halogen, an azide group, a hydroxy group, a nitro group, an amino group, a formyl group, a cyano group or one or more of two adjacent residues R are linked with each other to form an aromatic group, a cycloaliphatic group or a heterocyclic goup, and wherein n is an integer of 0 or 1 to 100 and preferably of 0 or 1 to 8, such as 0, 1, 2 or 3. Any of the unsubstituted and/or substitued C₁₋₂₀ hydrocarbon residues may be in particular a C₁₋₂₀-alkyl group, a C₁₋₂₀-alkenyl group, a C₁₋₂₀-alkynyl group, a C₁₋₂₀-alkoxy group, a C₄₋₂₀-cycloalkyl group, a C₅₋₂₀-aromatic group, a C₄₋₂₀-cycloaliphatic group or a C₅-₂₀-heterocyclic goup. One or more of two adjacent residues R may be in particular linked with each other to form a C₅₋₂₀-aromatic group, a C₄₋₂₀-cycloaliphatic group or a C₅₋₂₀-heterocyclic goup and preferably a C₅₋₇-aromatic group, a C₆₋₈₀-cycloaliphatic group or a C₅₋₇-heterocyclic goup.

Specific examples for suitable compounds according to the general formula (24) are those having any of the below general formulae (25) and (26): 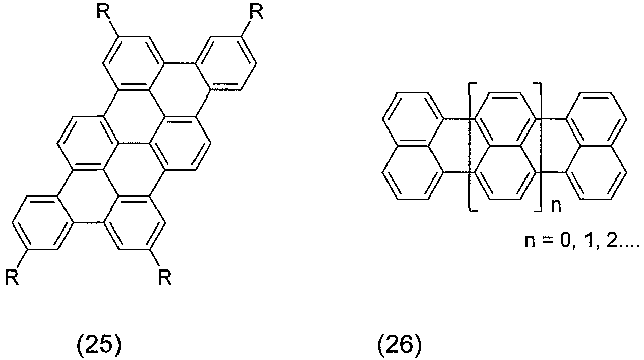 with R and n being as defined above.

In a further development of the idea of the present invention, it is suggested that the compound to be used comprises at least one heterocyclic ring group.

In accordance with a further particular embodiment of the present invention it is proposed that - as described above for the compounds having the general formula (2) - the compound to be used comprises at least one residue and preferably exactly one residue, which easily reacts with a respective coupling group at the target molecule. For instance, the compound to be used may have a residue with a terminal alkyne group. Such groups can be easily reacted for instance with an azide group of the target molecule, wherein the alkyne group and the azide group react under formation of a triazole group, which connects the fluorophore to the target molecule. Preferably, the compound to be used has one residue with a one terminal ethyne group.

Due to the aforementioned properties the compounds to be used in accordance with the present invention allows to obtain high resolution images with SMLM and STED having a resolution of as low as not more than 10 nm and preferably of less than 6 nm. On account thereof, the use in accordance with the present invention may be used for high resolution evaluations of biological systems, such as viruses or cells, in particular eukaryontic cells, bacterial cells, for the detection of material imperfections, such as micro- or nano-cracks, and for monitoring and detecting nano-structure fabrication and so on.

Subsequently, the present invention is further described by means of illustrative, but not limiting examples and figures.
- Fig. 1: shows a reaction scheme for synthesizing symmetric dibenzo[hi,st]ovalenes to be used in accordance with an embodiment of the present invention.
- Fig. 2: shows a reaction scheme for converting a symmetric dibenzo[hi,st]ovalene into an asymmetric dibenzo[hi,st]ovalene to be used in accordance with another embodiment of the present invention.
- Fig. 3a-d: show the absorption and emission spectra of four DBOV derivatives prepared in examples 2, 5, 7 and 8 (Abs: absorbance; PI: PL-emission).
- Fig. 4a-d: show the absorption and emission spectra of four polycyclic aromatic hydrocarbons shown in example 10 (Abs: absorbance; PI: PL-emission).
- Fig. 5a-b: show a widefield image (figure 5a) and a duty cycle diagram (figure 5b) showing the blinking properties of 6,14-bis(dimethylphenyl)dibenzo[hi,st]ovalene embedded in polystyrene.
- Fig. 6a-b: show a gravure printing plate (figure 6a), the surface of which has been analyzed with SMLM, and a respective image obtained for a section of this plate (figure 6a) and an enlarged cutout thereof (figure 6b).

### Example 1 - (Synthesis of 6,14-didodecyldibenzo[hi,st]ovalene)

### (Synthesis of 6, 6'-diiodo-[5, 5'-bichrysene]-3,3'-dicarbaldehyde)

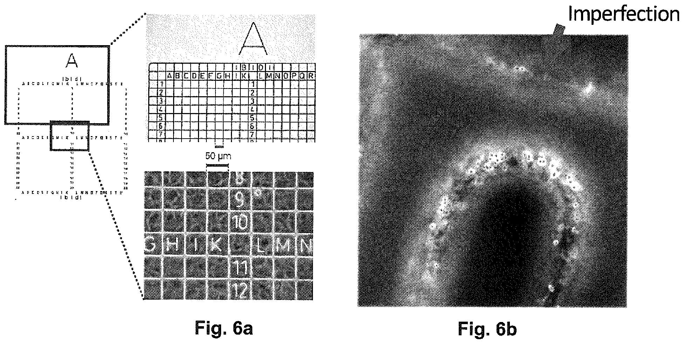

In accordance with the reaction scheme shown in figure 1, 6,6'-diiodo-[5,5'-bichrysene]-3,3'-dicarbaldehyde, which is compound 6 with all residues R₁ to R₇ being hydrogen atoms, was prepared starting from compound 1, in which all residues R₁ to R₃ are hydrogen atoms. Compound 1 was prepared as described in Nano Letters, 2017, volume 17, pages 5521 to 5525. Then, to a solution of compound 1 (2.0 g, 3.9 mmol) dissolved in anhydrous dichloromethane (240 mL) ICI (8.58 mmol, 8.58 mL, 1 M in dichloromethane) was added. After stirring at room temperature for 2 hours, the excess ICI was quenched by addition of saturated aqueous Na₂S₂O₃ solution (50 mL). The organic phase was separated, washed with brine (50 mL), dried over Na₂SO₄ and evaporated. The residual solid was recrystallized with dichloromethane and methanol.

After filtration, the product (2.2 g, 76%) was obtained as white solid. The product had the following characteristics:
Mp: >400 °C; ¹H NMR (300 MHz, Methylene Chloride-*d*₂) δ 9.18 (d, *J* = 9.2 Hz, 2H), 9.00 (d, *J* = 8.5 Hz, 2H), 8.72 (s, 2H), 8.53 - 8.42 (m, 4H), 8.28 (d, *J* = 9.1 Hz, 2H), 8.04 (d, *J* = 8.3 Hz, 2H), 7.98 - 7.88 (m, 2H), 7.86 - 7.74 (m, 4H); ¹³C NMR (75 MHz, methylene chloride-*d*₂) δ 191.8, 150.1, 137.4, 135.3, 134.6, 134.5, 133.7, 132.1, 130.9, 130.5, 130.4, 130.2, 129.7, 129.5, 129.3, 125.7, 124.4, 123.7, 114.1; FD-MS (8 kV): m/z 762.2; HRMS (MALDI-TOF): m/z Calcd for C₃₈H₂₀I₂O₂: 761.9553 [M]+, found: 761.9553 (error = 0 ppm).

Thus, it was shown that the product had the formula:

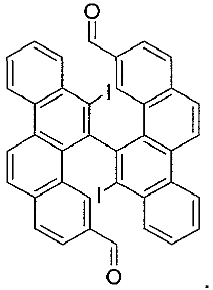

### (Synthesis of 5,14-diformylbenzo[a]dinaphtho[2,1,8-cde:1',2',3',4'-ghi]perylene)

In accordance with the reaction scheme shown in figure 1, 5,14-diformylbenzo[a]dinaphtho[2,1,8-cde:l',2',3',4'-ghi]perylene, which is compound 7 with all residues R₁ to R₇ being hydrogen atoms, was prepared starting from compound 6 prepared as described above. More specifically, to a 3 L cylindrical quartz reactor containing 6,6'-diiodo-[5,5'-bichrysene]-3,3'-dicarbaldehyde (300 mg, 0.394 mmol) was added a mixture of acetone (600 mL) and triethylamine (6 mL). Then the mixture was degassed by bubbling with Ar for 20 minutes. After that, the reaction mixture was stirred and irradiated at room temperature in a photoreactor equipped with six 300 nm wavelength UV lamps with strong stirring for 2 hours.

After cooling down to room temperature, the solvent was evaporated and the residue was purified by column chromatography (*n*-hexane:ethyl acetate = 4:1) to give the product (170 mg, 86% yield) as red solid. The product had the following characteristics, which was further confirmed by X-ray single crystal structure analysis. Mp: >400 °C; ¹H NMR (300 MHz, 1,1,2,2-tetrachloroethane-d₂) *δ* 9.46 (s, 2H), 9.01 (d, *J* = 9.1 Hz, 2H), 8.90 (d, *J* = 8.4 Hz, 2H), 8.52 - 8.42 (m, 4H), 8.34 (t, *J* = 9.1 Hz, 4H), 7.83 - 7.72 (m, 2H), 7.64 (dd, *J* = 8.1, 1.1 Hz, 2H); ¹³C NMR (75 MHz, C₂D₂Cl₄) *δ* 190.8, 133.7, 131.7, 131.6, 130.6, 128.5, 128.2, 127.9, 127.5, 127.1, 127.1, 125.9, 124.9, 124.4, 124.0, 123.9, 123.2, 121.2, 120.6; FD-MS (8 kV): m/z 506.9; HR MS (MALDI-TOF): *m*/*z* Calcd for C₃₈H₁₈O₂: 506.1307 [M]+, found: 506.1288 (error = -3.7 ppm).

Thus, it was shown that the product had the formula:

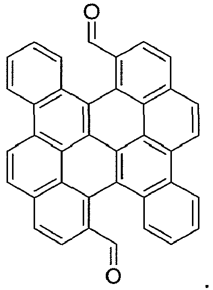

### (Synthesis of 6, 14-didodecyldibenzo[hi,st]ovalene)

In accordance with the reaction scheme shown in figure 1, 4,12-didodecyldibenzo[hi,st]ovalene, which is compound 9 with all residues R₁ to R₇ being hydrogen atoms and with residues Ar being n-dodecyl, was prepared starting from compound 7 prepared as described above. More specifically, to a 50 mL round bottom flask was added 5,14-diformylbenzo[a]dinaphtho[2,1,8-cde:1',2',3',4'-*ghi*]perylene (10.1 mg, 0.0199 mmol), the flask was evacuated and backfilled with Ar for 3 times before dry tetrahydrofuran (10 mL) was added. To the mixture was injected a solution of *n*-C₁₂H₂₅MgBr (0.30 mmol, 0.30 mL, 1N) in ether. After stirring at room temperature for 5 hours, the reaction was quenched by addition of saturated NH₄Cl solution (10 mL). The organic phase was extracted with ethyl acetate (15 mL) for three times, washed with brine (30 mL) dried over Na₂SO₄ and evaporated. After drying under vacuum for 2 hours, the residue was dissolved in dry dichloromethane (10 mL) and degassed by bubbling with dichloromethane saturated Ar flow for 10 minutes. BF₃•OEt₂ (0.1 mL) was added and the mixture was stirred overnight at room temperature. After quenching with methanol (1 mL), *p*-chloranil (10 mg, 0.041 mmol) was added and stirred for 2 hours.

The crude product was precipitated by addition of methanol (30 mL), which was further purified by column chromatography (*n*-hexane:tetrahydrofuran = 4:1 to 0:1) to give the product (6.5 mg, 41% yield) as blue solid. The product had the following characteristics:
Mp: >400 °C. HRMS (MALDI-TOF): m/z Calcd for C62H64: 808.5008 [M]+, found: 808.4978 (error = -3.7 ppm).

Thus, it was shown that the product had the formula:

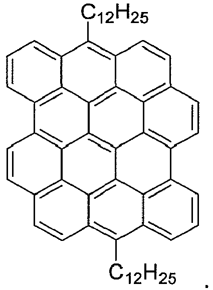

The maximum absorption wavelength in toluene (10⁻⁵ mol/L) was 611 nm (molar extinction coefficient ε = 2.03×10⁵ M⁻¹ cm⁻¹) with a high fluorescence quantum yield of 0.85.

### Example 2 - (Synthesis of 6,14-bis(dimethylphenyl)dibenzo[hi,st]ovalene [DBOV-DMEP])

In accordance with the reaction scheme shown in figure 1, 6,14-bis(dimethylphenyl)dibenzo[hi,st]ovalene, which is compound 9 shown in figure 1 with all residues R₁ to R₇ being hydrogen atoms and with residues Ar being 2,6-dimethylenpehnyl, was prepared starting from compound 7 prepared as described in example 1. More specifically, to a solution of 5,14-diformylbenzo[*a*]dinaphtho[2,1,8-*cde*:l',2',3',4'-*ghi*]perylene prepared as described in example 1 (63 mg, 0.12 mmol) dissolved in dry THF (63 mL) was added 2,6-dimethylphenylmagnesium bromide solution (1.8 mmol, 1.8 mL, 1N in THF). After stirring for 3 hours at room temperature, the reaction was quenched with saturated NH₄Cl solution (45 mL). The mixture was extracted with ethyl acetate (50 mL) for three times and the organic solution was combined, washed with brine (30 mL), dried over Na₂SO₄ then evaporated. After drying under vacuum for 2 hours, the residue was dissolved in anhydrous dichloromethane (60 mL) and degassed with dichloromethane vapor saturated argon flow. BF₃·OEt₂ (0.6 mL) was added and the reaction mixture was stirred overnight. After quenching with methanol (2 mL), *p-*chloranil (30 mg, 0.12 mmol) was added and the mixture was stirred for 2 hours at room temperature.

The solvent was evaporated and the residue was purified by column chromatography (*n*-hexane:DCM = 3:1) to give the product (59 mg, 72% yield) as blue solid. The product had the following characteristics:
Mp: >400 °C; ¹H NMR (300 MHz, THF-*d*₈) *δ* 9.54 (d, *J* = 8.3 Hz, 2H), 9.23 (d, *J* = 7.7 Hz, 2H), 8.59 (d, *J* = 8.2 Hz, 2H), 8.13 (d, *J* = 9.2 Hz, 2H), 8.00 (t, *J* = 7.9 Hz, 2H), 7.87 (d, *J* = 8.1 Hz, 2H), 7.70 (d, *J* = 9.1 Hz, 2H), 7.45 (dd, *J* = 6.1, 2.7 Hz, 4H), 7.40 (s, 3H), 2.00 (s, 12H); ¹³C NMR (75 MHz, THF-*d*₈) *δ* 138.8,138.7,135.9,133.2, 131.8, 131.5, 130.6, 129.9, 129.9, 129.1, 128.9, 128.8, 128.1, 127.0, 126.9, 126.1, 125.5, 125.4, 124.1, 124.1, 122.5, 121.9, 20.9; HRMS (MALDI-TOF): m/z Calcd for C54H32: 680.2504 [M]+, found: 680.2487 (error = -2.5 ppm).

Thus, it was shown that the product had the formula:

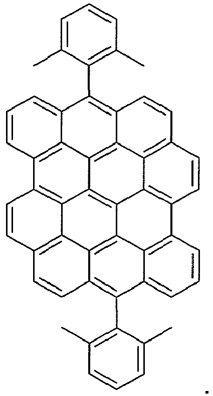

It was evaluated that this compound has a narrow absorption spectrum as well as a narrow emission spectrum, which are both shown in figure 3a (Abs: absorbance; PI: PL-emission).

The maximum absorption wavelength in toluene (10⁻⁵ mol/L) was 609 nm (molar extinction coefficient ε = 2.83×10⁵ M⁻¹ cm⁻¹)with a high fluorescence quantum yield of 0.85.

### Example 3 - (Synthesis of triisopropylsilyl ethynyl substituted 6,14-dimesityldibenzo[hi,st]ovalene)

### (Synthesis of dibrominated 6,14-dimesityldibenzo[hi,st]ovalene)

To a solution of 6,14-dimesityldibenzo[hi,st]ovalene (14 mg, 0,020 mmol) dissolved in tetrahydrofuran (70 mL) was added NBS (14 mg, 0,079 mmol). The resulting mixture was stirred at room temperature for 2 h. The solvent was evaporated and the residue was purified by column chromatography to give the product (14 mg, 84%) as blue solid. The product had the following characteristics:
Mp: >400 °C; ¹H NMR (700 MHz, THF-*d₈*) δ 10.67 (d, *J* = 8.3 Hz, 2H), 8.72 (d, *J* = 8.5 Hz, 2H), 8.34 (d, *J* = 8.8 Hz, 2H), 8.29 (d, *J* = 9.2 Hz, 2H), 7.87 (d, *J* = 9.0 Hz, 2H), 7.85 (d, *J* = 8.8 Hz, 2H), 7.27 (s, 4H), 1.93 (s, 12H); MS (MALDI-TOF): m/z Calcd for C54H30Br2: 864.10 [M]+, found: 864.06.

Thus, it was shown that the product had the formula:

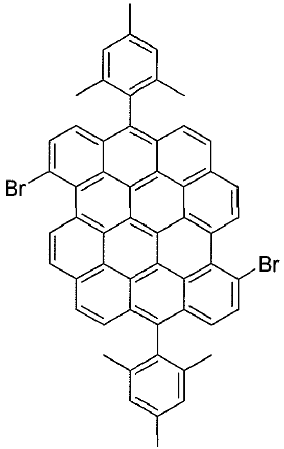

### (Synthesis of triisopropylsilyl ethynyl substituted 6,14-dimesityldibenzo[hi,st]ovalene)

To a Schlenk tube equipped with a stirring bar was added 3,11-dibromo-6,14-dimesityldibenzo[hi,st]ovalene prepared above (4.3 mg, 5.0 µmol), Pd(PPh3)4 (1.2 mg, 1.0 µmol) and Cul (0.38 mg, 2.0 µmol). The reaction tube was evacuated and backfilled with Argon for three times before addition of tetrahydrofuran (3 mL) and triethyl amine (1 mL). After degassing by three times freeze-pump-thaw cycles, triisopropylsilyl ethynyl (TIPS) acetylene (4.5 mg, 25 µmol) was added using a syringe. The resulting mixture was heated at 80 °C for 16 h.

After cooling down to room temperature, the solvent was evaporated and the residue was purified by column chromatography (*n*-hexane : ethyl acetate = 10:1) to give product (4.0 mg, 75%) as blue solid. The product had the following characteristics:
MS (MALDI-TOF): m/z Calcd for C78H76Si2: 1068.55 [M]+, found: 1068.55

Thus, it was shown that the product had the formula:

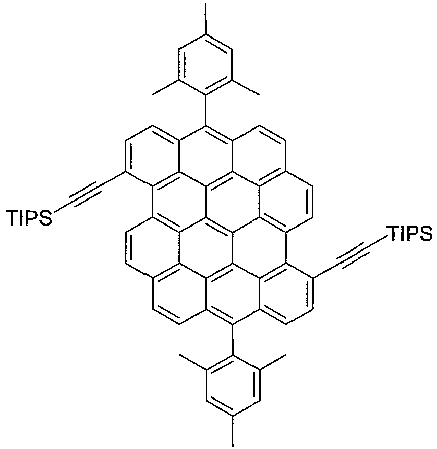

### Example 4 - (Synthesis of 3,11-bis(3,4,5-tris(2,5,8,11-tetraoxatridecan-13-yloxy)phenyl)-6,14-dimesityldibenzo[hi,st]ovalene)

To a Schlenk tube equipped with a stirring bar was added 3,11-dibromo-6,14-dimesityldibenzo[hi,st]ovalene prepared as described in example 3 (3.0 mg, 3.46 µmol), 3,4,5-tris(tetraethylene glycol monomethyl ether)phenyl boronic acid pinacol ester (12 mg, 14 µmol), Pd(PPh₃)₄ (1.6 mg, 1.4 µmol) and K₂CO₃ (4,8 mg, 35 µmol). The reaction tube was evacuated and backfilled with Ar for three times before a mixture of toluene/EtOH/H₂O = 2 mL/0.5 mL/0.5 mL was added. The mixture was degassed by three time freeze-pump-thaw cycles and heated at 90 °C overnight. After cooling down to room temperature, the reaction solution was extracted with ethyl acetate, washed with brine, dried over Na₂SO₄ and evaporated.

The residue was purified by column chromatography (ethyl acetate : MeOH = 10 :1 to 1:1) to give the product (5 mg, 69%) as blue oil. The product had the following characteristics:
¹H NMR (250 MHz, THF-*d₈*) δ 8.95 (d, *J* = 8.6 Hz, 1H), 8.24 (d, *J* = 8.7 Hz, 1H), 8.09 (d, *J* = 9.3 Hz, 1H), 7.95 (s, 2H), 7.73 (d, *J* = 9.2 Hz, 1H), 7.26 (s, 2H), 7.01 (s, 2H), 4.31 (t, *J* = 5.2 Hz, 2H), 3.93 - 3.84 (m, 2H), 3.75 (d, *J* = 5.5 Hz, 8H), 3.68 - 3.31 (m, 84H), 3.27 (s, 8H), 3.19 (s, 6H), 1.96 (s, 6H). MS (MALDI-TOF): m/z Calcd for C122H152030: 2097.04 [M]+, found: 2097.06

Thus, it was shown that the product had the formula:

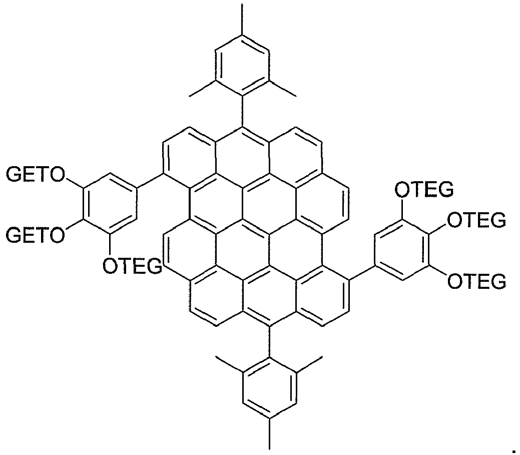

The solubility of this compound in water was evaluated at room temperature and was found to be 0.1 g/l.

### Example 5 - (Synthesis of 6,14-di(triisopropylsilyl ethynyl) dibenzo[hi,st]ovalene [DBOV-TIPS])

To an oven dried 25 mL Schlenk tube was added tetrahydrofuran (1.4 mL) and (triisopropylsilyl)acetylene (182 mg, 0.998 mmol). The solution was cooled down to 0°C and *n*-BuLi (0.6 mL, 0.96 mmol, 1.6 N in *n*-hexane) was added slowly. The mixture was stirred at room temperature for 30 mins and the resulting solution (1.5 mL, 0.73 mmol) was transferred to a solution of 5,14-diformylbenzo[*a*]dinaphtho[2,1,8-*cde*:1',2',3',4'-*ghi]*perylene prepared as described in example 1 (25 mg, 0.049 mmol) in dry THF (25 mL). After stirring for 21 h, the reaction was quenched by addition of saturated aqueous solution of NH₄Cl (20 mL) and extracted with ethyl acetate (20 mL) for three times. The combined organic phase was washed with brine (20 mL), dried over Na₂SO₄ and evaporated. The residue was dissolved in anhydrous dichloromethane (25 mL) and degassed with dichloromethane vapor saturated argon flow. After addition of BF₃·OEt₂ (0.025 mL), the resulting solution was stirred at room temperature for 6 h. The reaction was quenched by addition of methanol (1 mL) and *p*-chloranil (12 mg, 0.049 mmol) was added. The mixture was stirred for 3 h and the solvent was evaporated.

The residue was purified by recrystallization from dichloromethane and methanol to give the product (10 mg, 50% yield) as blue solid. The product had the following characteristics:
Mp: >400 °C. HRMS (MALDI-TOF): m/z Calcd for C60H56Si2: 832.3921 [M]+, found: 832.3859 (error = -7.4 ppm).

Thus, it was shown that the product had the formula:

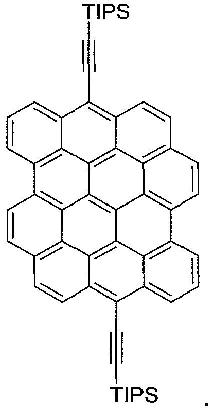

It was evaluated that this compound has a narrow absorption spectrum as well as a narrow emission spectrum, which are both shown in figure 3b (Abs: absorbance; PI: PL-emission).

The maximum absorption wavelength in toluene (10⁻⁵ mol/L) was 647 nm (molar extinction coefficient ε = 61511 M⁻¹ cm⁻¹)with a fluorescence quantum yield of 0.67.

### Example 6 - (Synthesis of 6,14-bis[3,4,5-tris(dodecoxyl)-phenyl]dibenzo[hi,st]ovalene)

### (Synthesis of 5-bromo-1,2,3-tris(dodecyloxy)benzene)

5-Bromo-1,2,3-trimethoxybenzene (2.5 g, 10 mmol) was dissolved in dry DCM (30 mL), the temperature was cooled down to -78 °C and stirred for 10 minutes before BBr₃ (8.26 g, 33.0 mmol) was added dropwise. After addition, the reaction mixture was gradually warmed up to room temperature and stirred overnight. The reaction mixture was added into a 100 mL of ice water and then extracted with ethylacetate (100 mL) for three times. The organic phase was combined, washed with brine (100 mL) and dried over Na₂SO₄. After evaporation of the solvent under reduced pressure and dried under vacuum pump for 2 hours, crude 5-bromobenzene-1,2,3-triol (2.0 g, 98%) was obtained as white solid. This intermediate was used directly for the next step without characterization and further purification.

To a 100 mL Schlenk flask was added 5-bromobenzene-1,2,3-triol (2.0 g, 9.8 mmol), 1-bromododecyl (9.92 g, 40.0 mmol) and K₂CO₃ (5.52 g, 40.0 mmol). The flask was evacuated and backfilled with Ar for three times before N,N-dimethylformamide (50 mL) was added. The mixture was heated at 80 °C for 20 hours. After completion of the reaction shown by TLC (*n*-hexane:ethyl acetate = 10:1), the mixture was cooled down to room temperature and diluted with ethyl acetate (200 mL), washed with water (50 mL), brine (50 mL), dried over Na₂SO₄ and evaporated.

The obtained residue was purified by column chromatography (n-hexane) and recrystallized with ethanol to give 5-bromo-1,2,3-tris(dodecyloxy)benzene (4.5 g, 63% yield). The product had the following characteristics:
Mp: >400 °C; ¹H NMR (300 MHz, Methylene Chloride-*d*₂) *δ* 6.68 (s, 2H), 3.97 - 3.83 (m, 6H), 1.85 - 1.72 (m, 4H), 1.72 - 1.62 (m, 2H), 1.51 - 1.39 (m, 6H), 1.39 - 1.22 (m, 48H), 0.94 - 0.82 (m, 9H); ¹³C NMR (75 MHz, Methylene Chloride-d2) *δ* 154.3, 137.8, 115.8, 110.3, 73.8, 69.7, 32.4, 30.7, 30.2, 30.2, 30.1, 30.1, 30.1, 30.0, 29.8, 29.8, 29.7, 26.5, 26.5, 23.1, 14.3; FD-MS (8 kV): m/z 709.6; HRMS (MALDI-TOF): m/z Calcd for C42H77BrO3: 708.5056 [M]+, found: 708.5005 (error = -6.5 ppm).

### (Synthesis of 6, 14-bis[3,4,5-tris(dodecoxyl)phenyl]dibenzo[hi,st]ovalene)

To a 25 mL Schlenk tube was added magnesium turnings (27 mg, 1.1 mmol), I₂ (3 mg, 0.011 mmol) and tetrahydrofuran (1 mL). The mixture was gently heated while approximately 1 mL of 5-bromo-1,2,3-tris(dodecyloxy)benzene (568 mg, 0.8 mmol) dissolved in tetrahydrofuran (3 mL) was added. As soon as the solution became colorless, the remaining solution was added dropwise under mild reflux and stirring was continued overnight to give Grignard reagent solution. The Grignard reagent solution (4 mL) prepared above was transferred to a solution of 5,14-diformylbenzo[*a*]dinaphtho[2,1,8-cde:1',2',3',4'-*ghi*]perylene prepared as described in example 1 (25 mg, 0.049 mmol) in dry THF (30 mL). After stirring at room temperature for 4 h, the reaction was quenched by addition of saturated NH₄Cl solution (15 mL). After stirring for 15 mins, the solution was extracted with ethyl acetate (50 mL) for three times. The combined organic phase was washed with brine, dried with Na₂SO₄ and evaporated.

The residue obtained above was dried under vacuum for 2 h and dissolved in anhydrous dichloromethane (30 mL). After bubbling with dichloromethane vapor saturated argon flow for 15 mins, BF₃·OEt₂ (0.1 mL) was added and the stirring was continued overnight. After quenching with methanol (2 mL), p-chloranil (12 mg, 0.049 mmol) was added and the mixture was stirred for 2 hours at room temperature.

The solvent was evaporated and the residue was purified by column chromatography (n-hexane:DCM = 3:1 to 0:1) to give the product (72 mg, 85% yield) as blue solid. The product had the following characteristics:
Mp: >400 °C; ¹H NMR (300 MHz, THF-*d*₈) *δ* 9.27 (d, *J* = 8.5 Hz, 2H), 9.02 (d, *J* = 7.7 Hz, 2H), 8.36 (d, *J* = 8.4 Hz, 2H), 8.10 (d, *J* = 8.3 Hz, 2H), 7.97 (t, *J* = 9.1 Hz, 4H), 7.89 (d, *J* = 9.3 Hz, 2H), 6.87 (s, 4H), 4.16 (t, *J* = 6.2 Hz, 4H), 4.05 (t, *J* = 6.2 Hz, 8H), 1.97 - 1.79 (m, 15H), 1.69 - 1.61 (m, 4H), 1.61 - 1.15 (m, 119H), 0.97 - 0.80 (m, 18H); ¹³C NMR (75 MHz, THF-*d*₈) *δ* 139.1, 137.5, 134.9, 132.7, 132.5, 131.3, 130.3, 130.1, 128.9, 127.9, 127.5, 127.3, 126.5, 125.5, 125.1, 124.7, 124.3, 123.6, 123.4, 122.1, 121.4, 111.0, 74.0, 70.0, 33.2, 33.1, 31.9, 31.1, 31.1, 31.1, 31.0, 30.9, 30.9, 30.8, 30.7, 30.7, 30.6, 27.6, 27.4, 23.9, 23.9, 14.9, 14.9; HRMS (MALDI-TOF): m/z Calcd for C122H16806: 1729.2841 [M]+, found: 1729.2822 (error = -1.1 ppm).

Thus, it was shown that the product had the formula:

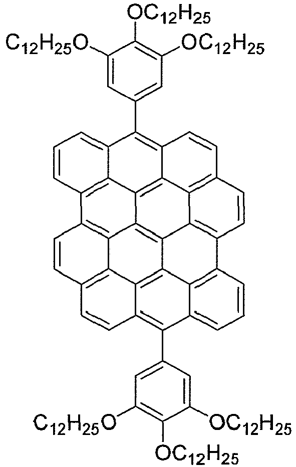

The maximum absorption wavelength in toluene (10⁻⁵ mol/L) was 609 nm (molar extinction coefficient ε = 3.85×10⁵ M⁻¹ cm⁻¹) with a high fluorescence quantum yield of 0.89.

### Example 7 - (Synthesis of 6,14-diphenyldibenzo[hi,st]ovalene [DBOV-Ph])

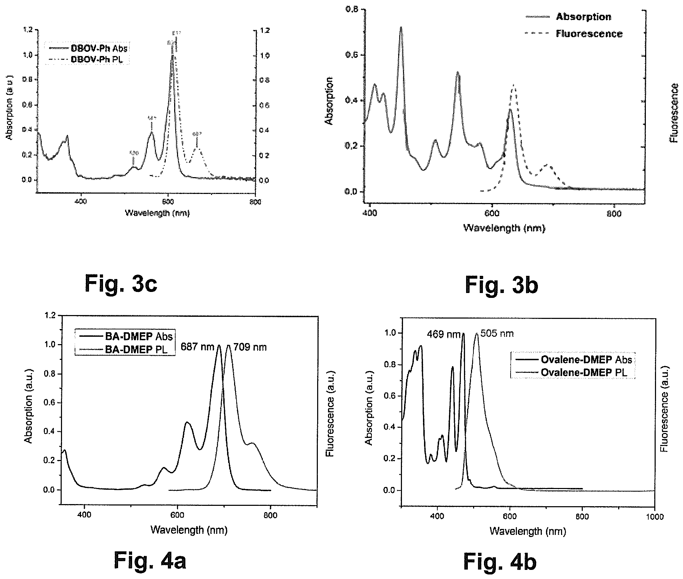

6,14-diphenyldibenzo[hi,st]ovalene was prepared analogous to example 2 by using phenylmagnesium bromide solution instead of 2,6-dimethylphenylmagnesium bromide solution. It was evaluated that this compound has a narrow absorption spectrum as well as a narrow emission spectrum, which are both shown in figure 3c (Abs: absorbance; PI: PL-emission).

### Example 8 - Synthesis of N-hexyl fumaric acid imide group substituted 6,14-dimesityldibenzo[hi,st]ovalene)

6,14-dimesityldibenzo[hi,st]ovalene was reacted with N-hexyl fumaric acid imide in diphenylether for 2 days at 275°C. The analysis showed that the product had the chemical formula C₇₆H₅₈N₂O₄ with a measured mass of 1.062,4324 g/mol. Thus, the formula was confirmed to be:

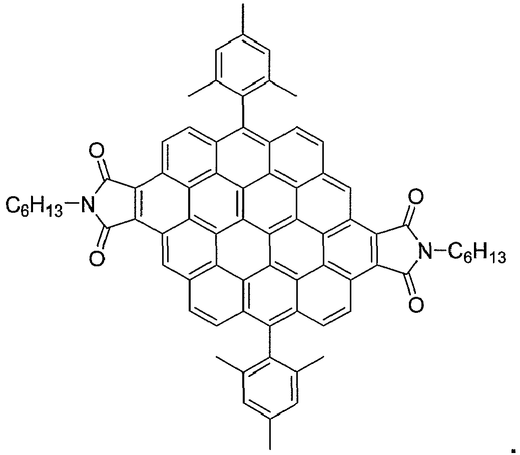

It was evaluated that this compound has a narrow absorption spectrum as well as a narrow emission spectrum, which are both shown in figure 3d.

### Example 9 - Toxicity of 3,11-bis(3,4,5-tris(2,5,8,11-tetraoxatridecan-13-yloxy)phenyl)-6,14-dimesityldibenzo[hi,st]ovalene)

3,4,5-Tris(hexaethylene glycol) substituted 6,14-dimesityldibenzo[hi,st]ovalene was prepared analogous to example 4 and evaluated concerning its toxicity. It was found that the compound is non-toxic up to a concentration of 5 µM, which is a sufficiently high concentration in terms of bioimaging. The compound has a good solubility in DMSO.

### Example 10 - Spectral properties of other polycyclic aromatic hydrocarbons

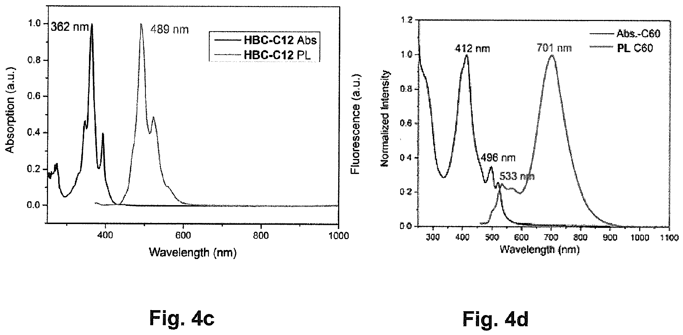

The absorption and emission spectra of the following four polycyclic aromatic hydrocarbons have been recorded, which are shown in figures 4a to 4d.

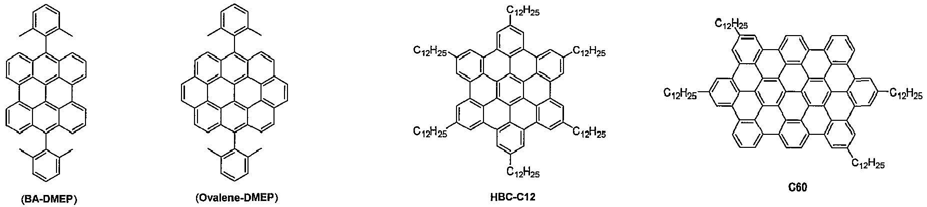

The absorption and emission spectra of all these compounds have been evaluated and were sufficiently narrow for both, SMLM and STED microscopy. The respective spectra are shown in figures 4a to 4d (Abs: absorbance; PI: PL-emission).

### Example 11 - Evaluating the suitability for SMLM / Performing SMLM measurement

Different measurements were performed with 6,14-dimesityldibenzo[hi,st]ovalene being embedded in a polystyrene matrix and others with 6,14-dimesityldibenzo[hi,st]ovalene in air. A Leica GSD microscopy was used for the microscopy with an imaging laser wavelength: of 532 nm.

### (Sample Preparation: Embedding with polystyrene)

6,14-dimesityldibenzo[hi,st]ovalene powder was dispersed in toluene at room temperature. The diluted solution having a concentration of e.g. 10⁻⁸ mol/L, 10⁻⁹ mol/L, 10⁻¹⁰ mol/L or 10⁻¹¹ mol/L, respectively, was mixed with the same amount of toluene solution (0.08 mg/mL) of polystyrene.

A glass coverslip (#1.5) was cleaned by oxygen-plasma cleaner (250 W, 5 minutes). Approximately 1 µl of the GQD-PS solution was spin-coated on the oxygen-plasma-treated glass coverslip for 60 s, namely for 20 s at 2,000 rpm and for 40 s at 4,000 rpm.

The sample was dried by heating to 90°C for 1 h on a hot plate.

The prepared glass coverslip was taped to a slide and put on the microscope for imaging test.

### (Sample Preparation: In air environment)

6,14-dimesityldibenzo[hi,st]ovalene powder was dispersed in toluene at room temperature to a concentration of 10⁻⁵ mol/L and was then further diluted to the needed concentration of e.g. 10⁻⁸ mol/L, 10⁻⁹ mol/L, 10⁻¹⁰ mol/L or 10⁻¹¹ mol/L, respectively.

A glass coverslip (#1.5) was cleaned by oxygen-plasma cleaner (250 W, 5 minutes).

About 10 µl solution were put on top of the cleaned coverslip and then covered by a glass slide. It was waited until the whole toluene has volatilized completely and then the coverslip was taped to the slide. Afterwards, the prepared sample was put on the microscope for imaging testing.

### (Sample Preparation: For nano- and micro-structure imaging measurements)

The measured nano- and micro-structure sample was based on the gridded cover glass purchased from Ibidi GmbH in Martinsried, Germany. The nano structure was produced on this cover glass by Focus Ion Beam (FIB). For the sample preparation, 5 µl 10⁻⁹ mol/L solution were put on top of the micro/nano-structure sample. After a few minutes, when all of the toluene was volatilized completely, the prepared sample was put on the microscope for imaging testing.

### (Blinking behavior of 6,14-dimesityldibenzo[hi,st]ovalene embedded in polystyrene or in air)

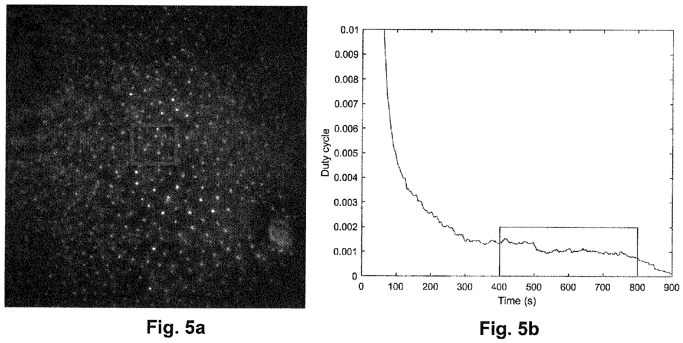

SMLM were performed with 6,14-dimesityldibenzo[hi,st]ovalene embedded in polystyrene or in air. 30 000 frames were recorded with an exposure time of 30 ms each within 30 min. Figure 5a shows a widefield image of the sample and figure 5b shows a part of the duty cycle diagram. It was thus confirmed that 6,14-dimesityldibenzo[hi,st]ovalene is well suited for SMLM.

### (Surface detection by SMLM)

A gridded cover glass, whose surface provides 400 squarish recesses, wherein each recess has a cross-section of 50 µm x 50 µm and a depth of 5 µm, wherein some of the recesses contain numbers or letters, respectively, as shown in figure 6a. The sample was prepared as described above for the "Sample Preparation: For nano- and micro-structure imaging measurements". 30,000 frames were recorded with an exposure time of 30 ms each. Figures 6a and 6b show SMLM images of a section of the sample showing a surface imperfection in field with the number 10. As is can be seen in figures 6a and 6b the resolution of the SMLM images is so good that not only the surface structure is reproduced in detail, but so excellent that even small surface imperfections in form of a crack has been detected.

## Claims

1. Use of a compound in single-molecule localization microscopy (SMLM) or in stimulated emission depletion microscopy (STED), wherein the compound is a substituted or unsubstituted polycyclic aromatic hydrocarbon comprising six or more substituted and/or unsubstituted aromatic hydrocarbon rings, wherein each of at least six of the six or more substituted and/or unsubstituted aromatic hydrocarbon rings is fused with at least another one of the at least six substituted and/or unsubstituted aromatic hydrocarbon rings.

2. The use in accordance with claim 1, wherein the compound is used in the high-resolution microscopy as fluorescent marker, and wherein the compound is used in photoactivated localization microscopy (PALM), stochastic optical reconstruction microscopy (STORM), ground state depletion individual molecule return (GSDIM), binding activated localization microscopy (BALM) or fluorescence photo-activation localization microscopy (FPALM).

3. The use in accordance with claim 1 or 2, wherein the compound comprises six to 91 substituted and/or unsubstituted aromatic hydrocarbon rings, wherein each of at least six and preferably each of all the six to 91 substituted and/or unsubstituted aromatic hydrocarbon rings is fused with at least another one of the at least six substituted and/or unsubstituted aromatic hydrocarbon rings.

4. The use in accordance with any of the preceding claims, wherein the compound comprises any of the below units:
i) -Ar₁(Ar₂)ₓ-, wherein the residues Ar₁ and Ar₂ are the same or different and independently from each other a substituted and/or unsubstituted aromatic hydrocarbon ring and x is in an integer of 5, 6 in the case that Ar₁ is at least a C₇-ring or 7,
ii) -Ar₁(Ar₂)ₓ-Ar₃(Ar₄)_{y}-, wherein the residues Ar₁ to Ar₄ are the same or different and independently from each other a substituted and/or unsubstituted aromatic hydrocarbon ring and x and y are independently from each other integers of 1 to 6, wherein the sum of x and y is at least 4, or
iii) -Ar₁(Ar₂)ₓ-Ar₃(Ar₄)_{y}-Ar₅(Ar₆)_{z}-, wherein the residues An to Ar₆ are the same or different and independently from each other a substituted and/or unsubstituted aromatic hydrocarbon ring and x, y and z are independently from each other integers of 1 to 7, wherein the sum of x, y and z is at least 3, or
iv) -Ar₁-(Ar₂)ₙ-Ar₃-, wherein the residues Ar₁ to Ar₃ are the same or different and independently from each other a substituted and/or unsubstituted aromatic hydrocarbon ring and n is an integer of 4 to 14, wherein the residues Ar₁ and Ar₃ may be bonded or fused with each other to form a ring.

5. The use in accordance with any of the preceding claims, wherein the compound comprises a unit with the general formula (1) or the compound has the general formula (1): 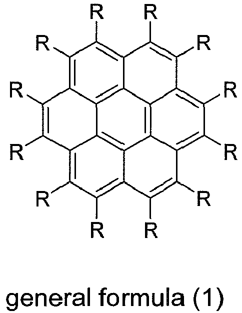 wherein in general formula (1) the residues R are the same or different and independently from each other a hydrogen atom, an unsubstituted C₁₋₂₀ hydrocarbon residue, a substitued C₁₋₂₀ hydrocarbon residue, a halogen, an azide group, a hydroxy group, a nitro group, an amino group, a formyl group, a cyano group or one or more of two adjacent residues R are linked with each other to form C₅₋₂₀-aromatic group, a C₄₋₂₀-cycloaliphatic group or a C₅₋₂₀-heterocyclic goup, wherein any of the unsubstituted and/or substitued C₁₋₂₀ hydrocarbon residues may be a C₁₋₂₀-alkyl group, a C₁₋₂₀-alkenyl group, a C₁₋₂₀-alkynyl group, C₁₋₂₀-alkoxy group, a C₄₋₂₀-cycloalkyl group, a C₅₋₂₀-aromatic group, a C₄₋₂₀-cycloaliphatic group or a C₅₋₂₀-heterocyclic goup.

6. The use in accordance with any of the preceding claims, wherein the compound comprises a unit with the general formula (2) or the compound has the general formula (2): 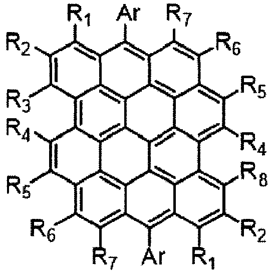 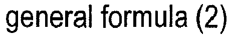 wherein in general formula (2):
residues R₁ to R₈ and Ar are independently from each other selected from the group consisting of hydrogen, unsubstituted alkyl groups, substituted alkyl groups, unsubstituted alkenyl groups, substituted alkenyl groups, unsubstituted alkynyl groups, substituted alkynyl groups, unsubstituted cycloalkyl groups, substituted cycloalkyl groups, unsubstituted aryl groups, substituted aryl groups, unsubstituted aralkyl groups, substituted aralkyl groups, unsubstituted hetaryl groups, substituted hetaryl groups, azide groups, and groups formed in that two of adjacent residues of R₁ to R₈ and Ar are linked with each other to form an aromatic, heteroaromatic, cyclic or heterocyclic group.

7. The use in accordance with claim 6, wherein in general formula (2) the residues R₁ to R₈ and Ar are independently from each other selected from the group consisting of hydrogen, unsubstituted linear or branched C₁₋₃₀-alkyl groups, unsubstituted C₃₋₃₀-cycloalkyl groups, phenyl groups, naphthyl groups, anthryl groups, pyrenyl groups, azide groups, polyethylene groups with 2 to 20 ethylene moieties, phenyl ethylene, triisopropylsilyl ethynyl, trimethylsilyl ethynyl, and groups formed in that two of adjacent residues of R₁ to R₈ and Ar are linked with each other to form an aromatic, heteroaromatic, cyclic or heterocyclic group, and preferably the residues R₁ to R₈ and Ar are independently from each other hydrogen, a C₁₋₂₀-alkyl group, a C₁₋₂₀-alkenyl group, a C₁₋₂₀-alkynyl group, a C₁₋₂₀-alkoxy group, a C₄₋₂₀-cycloalkyl group, a C₅₋₂₀-aromatic group, a C₄₋₂₀-cycloaliphatic group or a C₅₋₂₀-heterocyclic goup and two of adjacent residues of R₁ to R₈ and Ar may be linked with each other to form C₅₋₂₀-aromatic group, a C₄₋₂₀-cycloaliphatic group or a C₅₋₂₀-heterocyclic goup.

8. The use in accordance with claim 6 or 7, wherein in general formula (2):
residue R₁ is hydrogen or a C₁₋₂₀-alkyl group,
residues R₂ to R₈ are hydrogen and
residue Ar is aryl, a C₆₋₁₅-alkyl group or a trialkylsilyl alkynyl group.

9. The use in accordance with any of claims 6 to 8, wherein in general formula (2) residue Ar is selected from the group consisting of phenyl, trifluorphenyl, 1,5-dimethylphenyl, mesitylene, triisopropylsilyl ethynyl, trimethylsilyl ethynyl, phenylsilyl ethynyl and C₆₋₁₅-alkyl groups.

10. The use in accordance with any of claims 6 to 9, wherein in the general formula (2) at least one of the residues R₁ to R₈ and Ar is a hydrophilic group selected from the group consisting of groups comprising one or more carboxy groups, groups including one or more polyethylene residues with each 2 to 20 alkylene moieties and preferably 2 to 20 ethylene moieties, one or more sulfonate groups, one or more quaternary amine groups, one or more amide groups, one or more imine groups and one or more pyridine groups.

11. The use in accordance with any of claims 1 to 5, wherein the compound comprises a unit with the general formula (3) or the compound has the general formula (3): 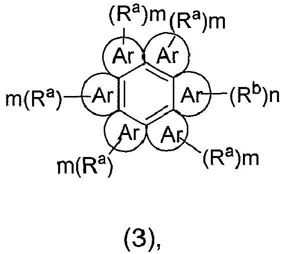 wherein in general formula (3) the residues R^{a} and R^{b} are the same or different and independently from each other a hydrogen atom, an unsubstituted C₁₋₂₀ hydrocarbon residue, a substitued C₁₋₂₀ hydrocarbon residue, a halogen, an azide group, a hydroxy group, a nitro group, an amino group, a formyl group, a cyano group or one or more of two adjacent residues R^{a} and R^{b} are linked with each other to form an aromatic group, a cycloaliphatic group or a heterocyclic goup, each of residues Ar is a cyclic group and m is an integer of 1 to 4.

12. The use in accordance with claim 11, wherein in the general formual (3) at least one of the residues Ar is a heterocyclic group and each of the remaining residues Ar is independently from each other a benzene group, a cycloaliphatic group or a heterocyclic goup.

13. The use in accordance with any of claims 1 to 4, 11 and 12, wherein the compound comprises a unit with the general formula (33) or the compound has the general formula (33): 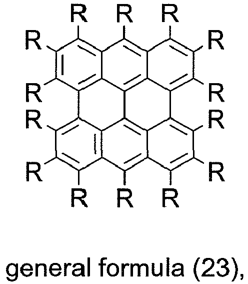 wherein in general formula (23) the residues R are the same or different and independently from each other a hydrogen atom, an unsubstituted C₁₋₂₀ hydrocarbon residue, a substitued C₁₋₂₀ hydrocarbon residue, a halogen, an azide group, a hydroxy group, a nitro group, an amino group, a formyl group, a cyano group or one or more of two adjacent residues R are linked with each other to form C₅₋₂₀-aromatic group, a C₄₋₂₀-cycloaliphatic group or a C₅₋₂₀-heterocyclic goup, wherein any of the unsubstituted and/or substitued C₁₋₂₀ hydrocarbon residues may be a C₁₋₂₀-alkyl group, a C₁₋₂₀-alkenyl group, a C₁₋₂₀-alkynyl group, a C₁₋₂₀-alkoxy group, a C₄₋₂₀-cycloalkyl group, a C₅₋₂₀-aromatic group, a C4-2o-cycloaliphatic group or a C₅₋₂₀-heterocyclic goup.

14. The use in accordance with any of the preceding claims, wherein the compound comprises a residue with a terminal alkyne group.

15. The use in accordance with any of the preceding claims for high resolution evaluations of biological systems, such as cells, for the detection of material imperfections, such as micro- or nano-cracks, and for monitoring and detecting nano-structure fabrication.
